# EUROPEAN PATENT SPECIFICATION

(11) **EP 1 346 034 B1**
(45) Date of publication and mention of the grant of the patent: **18.02.2009**
(21) Application number: 01997546.5
(22) Date of filing: 20.11.2001
(51) Int. Cl.: C12N 9/64, C12N 15/57, C07K 16/40, C12N 5/10, G01N 33/563, A61K 38/48

(54) **VON WILLEBRAND FACTOR (vWF) CLEAVING PROTEASE POLYPEPTIDE, NUCLEIC ACID ENCODING THE POLYPEPTIDE AND USE OF POLYPEPTIDE**
VON-WILLEBRAND-FAKTOR (vWF) SPALTENDES PROTEASE-POLYPEPTID, FÜR DAS POLYPEPTID CODIERENDE NUKLEINSÄURE UND VERWENDUNG DES POLYPEPTIDS
POLYPEPTIDE DE LA PROTEASE DE CLIVAGE DU FACTEUR DE VON WILLEBRAND (VWF), ACIDE NUCLEIQUE CODANT CE POLYPEPTIDE ET UTILISATION DE CE POLYPEPTIDE

(30) Priority: 22.11.2000 US 721254; 12.04.2001 US 833328
(43) Date of publication of application: 24.09.2003
(73) Proprietor: Baxter Innovations GmbH, 1221 Wien (AT)
(72) Inventor: LAEMMLE, Bernhard, CH-3065 Bolligen (CH); SCHALLER-GERRITSEN, Helena, Elisabeth, CH-5630 Muri AG (CH); FURLAN, Miha, CH-3006 Bern (CH); TURECEK, Peter, A-3400 Klosterneuburg (AT); SCHWARZ, Hans-Peter, A-1180 Vienna (AT); SCHEIFLINGER, Friedrich, A-1090 Vienna (AT); ANTOINE, Gerhard, A-2301 Gross-Enzersdorf (AT); KERSCHBAUMER, Randolf, A-3400 Klosterneuburg (AT); TAGLIAVACCA, Luigina, Piltello, 20093 Milano (IT); ZIMMERMANN, Klaus, A-3021 Pressbaum (AT); VOELKEL, Dirk, A-1220 Vienna (AT)
(74) Representative: Bassett, Richard Simon
(86) International application number: PCT/EP2001/013391
(87) International publication number: WO 2002/042441

(56) References cited:
- WO-A-97/41206
- FURLAN ET AL.: "Partial purification and characterization of a protease from human plasma cleaving the von Willebrand Factor to fragments produced by in vivo proteolysis" BLOOD, vol. 87, no. 10, 15 May 1996 (1996-05-15), pages 4223-4234, XP002042011
- TSAI ET AL. : "Antibodies to von Willeberand Factor-cleaving protease in acute thrombotic thrombocytopenic purpura" THE NEW ENGLAND JOURNAL OF MEDICINE, vol. 339, no. 22, 26 November 1998 (1998-11-26), pages 1558-1594, XP001079259
- FURLAN ET AL.: "Deficient activity of von Willebrand Factor-cleaving protease in chronic relapsing thrombocytopenic purpura" BLOOD, vol. 89, no. 9, 1 May 1997 (1997-05-01), pages 3097-3103, XP001070572
- GERRITSEN ET AL.: "Partial amino acid sequence of purified von Willebrand Factor-cleaving protease" BLOOD, vol. 98, no. 6, 15 September 2001 (2001-09-15), pages 1654-1661, XP002201351
- SOEJIMA ET AL.: "A novel human metalloprotease synthesized in the liver and secreted into the blood: possibly, the von Willebrand Factor-cleaving protease?" J. BOCHEM., vol. 130, October 2001 (2001-10), pages 475-480, XP001079224
- ZHENG ET AL.: "Structure of von Willebrand Factor-cleaving protease (ADAMTS13), a metallprotease involved in thrombotic thrombocytopenic purpura" J. BIOL. CHEM., vol. 276, no. 44, 2 November 2001 (2001-11-02), pages 41059-41063, XP002201352

## Description

### Field of the Invention

The invention relates to a vWF cleaving protease (vWF-cp) polypeptide or a partial sequence thereof, a nucleic acid molecule encoding the amino acid sequence of a vWF -cp polypeptide, and a composition comprising the polypeptide. The invention also relates to the use of the vWF-cp polypeptide for production of anti-vWF-cp polypeptide antibodies and for production of a preparation for prophylaxis and therapy of thrombosis and thromboembolic disease.

### Background of the Invention

vWF is a glycoprotein circulating in plasma as a series of multimers ranging in size from about 500 to 20,000 kD. Multimeric forms of vWF are composed of 250 kD polypeptide subunits linked together by disulfide bonds. vWF mediates the initial platelet adhesion to the subendothelium of a damaged vessel wall, though only the largest multimers appear to exhibit haemostatic activity. Such vWF multimers having large molecular masses are stored in the Weibel Palade bodies of endothelial cells, and it is believed that endothelial cells secrete these large polymeric forms of vWF. Those forms of vWF which have a low molecular weight (low molecular weight or LMW vWF) are believed to arise from proteolytic cleavage of the larger multimers.

A small portion of the vWF present in normal plasma circulates as 189, 176 and 140 kD fragments resulting from proteolytic degradation of vWF *in vivo,* the 140 kD fragment being derived from the N-terminal region, and the 176 kD fragment from the C-terminal region of the subunit. When low molecular weight (LMW) forms of vWF are isolated from normal human plasma and subjected to SDS-PAGE (polyacrylamide gel electrophoreses) after disulfide reduction, an unusually high portion of vWF fragments are found. This finding is compatible with the view that LMW forms of vWF have been partially or predominantly derived from large multimers by proteolytic degradation.

The proteolytic degradation of vWF is a physiological process in healthy individuals, yet in patients suffering from von Willebrand disease (vWD) type 2A it may be accelerated, and as a consequence these patients lack the vWF multimers with the largest molecular masses. A lack of large vWF multimers and an increased level of proteolytic fragments are also observed in acquired von Willebrand disease (vWD) associated with myeloproliferation syndrome, indicating increased *in vivo* proteolysis in this condition as well.

In patients with thrombotic thrombocytopenic purpura (TTP), on the other hand, unusually large vWF multimers are detected, and increased vWF binding to platelets has been demonstrated in these patients (Moake et al. 1982, N. Engl. J. Med. 307: 1432-1435). Familial TTP is associated with a severe congenital deficiency of vWF protease, while the presence of vWF-cleaving proteases inhibiting autoantibodies has been observed in patients with non-familial TTP.

The large multimers of vWF associated with TTP normally disappear after a patient is transfused with normal fresh frozen plasma. Presently, plasma exchange is the most important treatment for TTP, although significant side effects have been reported with this therapy. The existence of a severe congenital deficiency of vWF protease has been established in patients with familial TTP and the presence of a vWF-cleaving protease inhibiting autoantibodies has been observed in patients with non-familial TTP.

Several proteases have been shown to be able to cleave vWF, thereby impairing its binding affinity for platelets. However, in vitro the cleavage of vWF with these proteases in each case results in cleavage products different from the fragments derived from *in vivo* cleavage.

Thus, for example, while plasmin is capable of cleaving several peptide bonds in vWF, plasmin-treated vWF retains a high molecular weight core region retaining about 70% of its platelet agglutinating activity (determined as ristocetin cofactor). A 34 kD peptide is split from the N-termini of individual vWF subunits in the early stages of plasmin treatment, and epitope mapping of such plasmin-induced fragments show that these fragments originated from regions of the vWF subunit that are different from the vWF fragments present in circulating plasma.

Porcine pancreatic elastase and various serine proteases released from human leukocytes have also been shown to degrade vWF proteolytically with a resultant loss of large multimers. Epitope mapping of the degradation products again indicates that these fragments also differ from those present in normal plasma and in vWD type 2A. In addition, a calpain-like protease released from human platelets has been shown to degrade large vWF multimers and to create vWF fragments similar to those observed *in vivo*.

### Summary of the Invention

It is an object of the invention to provide for a vWF cleaving protease (vWF-cp) polypeptide or a partial sequence thereof.

It is another object of the invention to provide for a composition comprising a vWF-cp polypeptide.

It is an object of the present invention to provide for a nucleic acid molecule comprising a nucleic acid sequence encoding an amino acid sequence of a vWF-cp polypeptide.

It is also an object of the invention to provide for recombinant vWF-cp polypeptide.

It is another object of the invention to provide a method of production of a recombinant vWF-cp polypeptide.

It is also an object of the invention to provide for a method of purification of vWF using a vWF-cp polypeptide or a partial sequence thereof.

It is also an object of the invention to provide for anti-vWF-cp antibodies.

### Brief Description of the Drawing

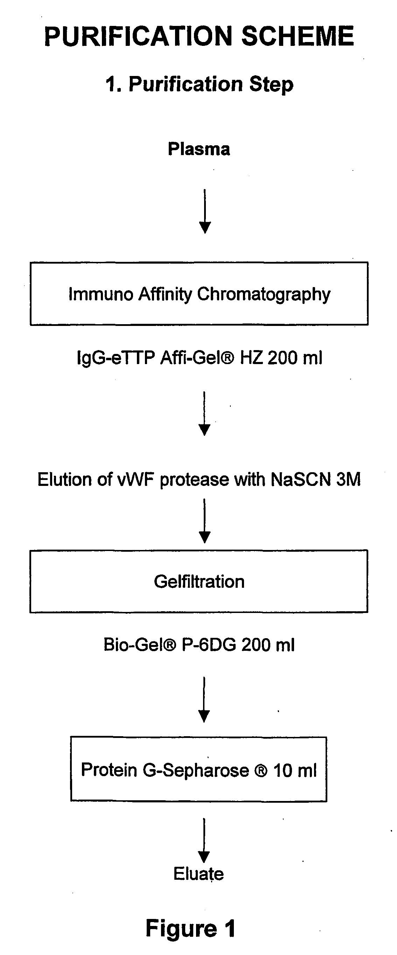

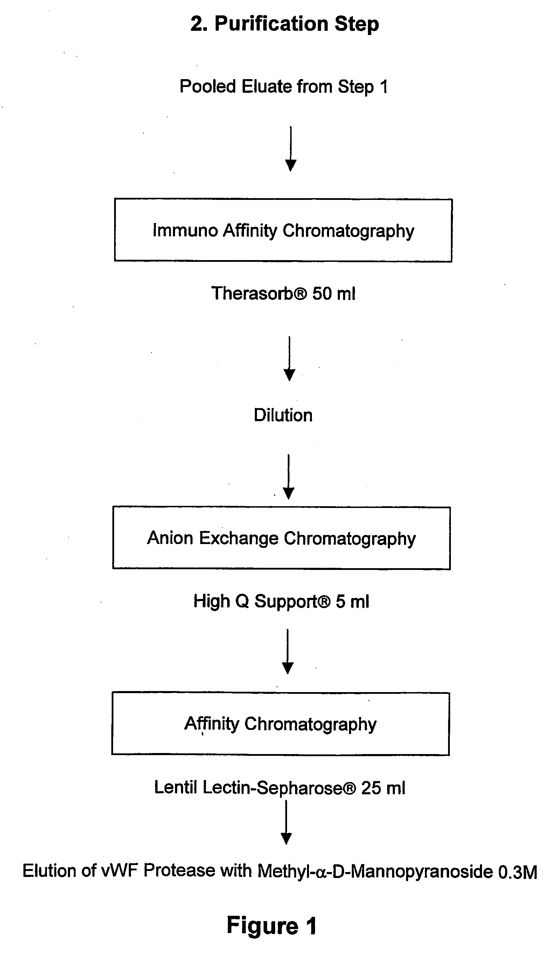

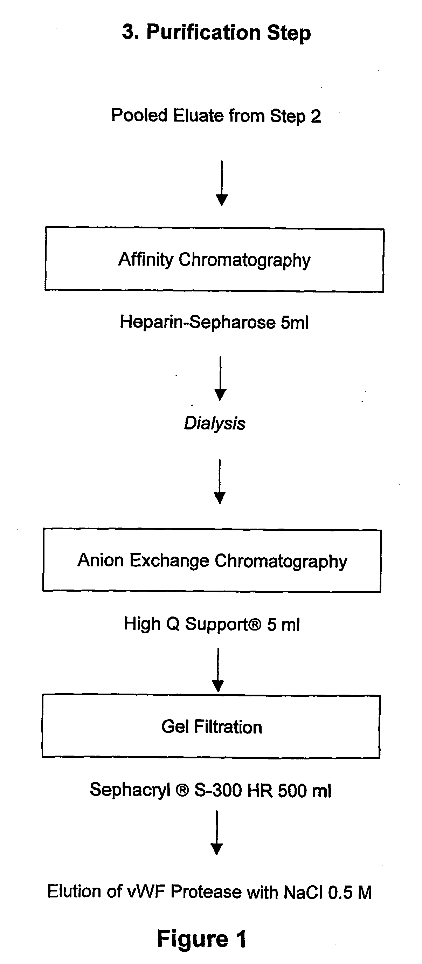

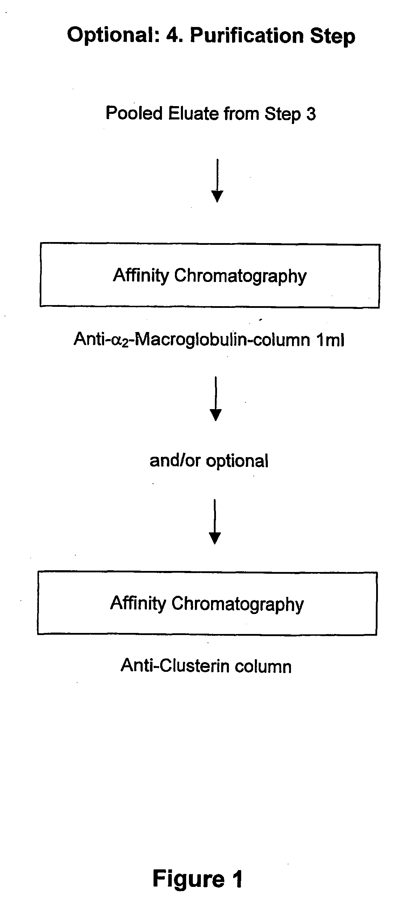

Fig.1 shows the schematic purification scheme of the vWF-cp from plasma.
Fig. 2 shows the SDS-PAGE of purified of vWF-cp polypeptides under non-reducing (A) and reducing conditions in the presence of DTT (B).

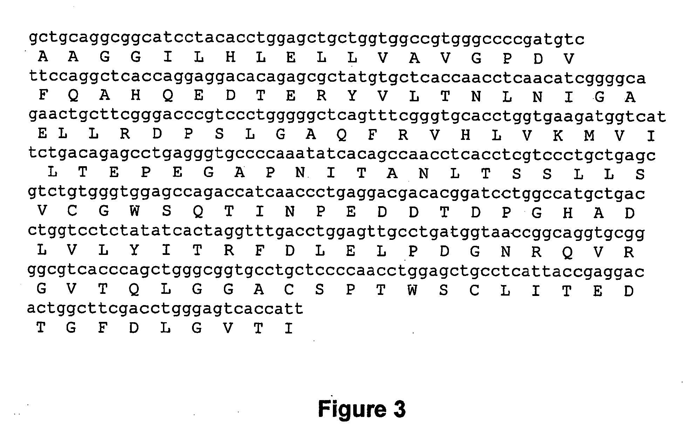

Fig. 3 shows the partial nucleotide and amino acid sequence of the vWF-cp polypeptide.
Fig. 4 shows the schematic drawing of the genomic localization of the vWF-cleaving protease gene.
Fig. 5 shows the complete amino acid and nucleotide sequence of the vWF-cleaving protease. Start and end of the signal peptide, metalloprotease, the cystein rich region (ACR), thromspondin type 1 motif (TSP 1), and disintegrin like motif (RGD) domains are indicated by arrows. Furin cleavage site, catalytic site and Met-turn are underlined. The putative N-glycosylation sites are indicated by asterisks.
Fig. 6 shows the schematic representation of the domain organization of the vWF-cleaving protease and those of the human members of the ADAM-TS family. The domain structures of the vWF-cleaving protease (ADAMTS 13) and of all known human ADAMTS proteins are shown. ADAMTS 10 cannot be found in databases and ADAMTS 11 is identical to ADAMTS 5.
Fig. 7 shows Western blot analysis of cells transfected with a vector comprising vWF-cp polypeptide encoding sequences, with lane 1: standard molecular weight marker, lane 2: control vector cDNA3.1 (+) and lane 3: vector pCMV-vWFcp. The specific vWF-cp polypeptide band is indicated by the arrow.
Fig. 8 shows SDS-Page of vWF-cp activity on vWF with lane 1: purified plasmatic vWF as control, lane 2: vWF incubated with normal human plasma, lane 3: vWF incubated with buffer as control, lane 4: vWF incubated with of cell lysate of SK-Hep cells transfected with control vector cDNA3.1 (+) and lane 5: vWF incubated with of cell lysate of SK Hep cells transfected with vWF-cp expressing vector pCMV-vWFcp.

### Detailed Description of the Invention

In accordance with one of the objects of the invention there is provided a polypeptide exhibiting a vWF-cp activity comprising the amino acid sequence SEQ ID. NO. 1, the vWF-cp activity being as defined in Claim 1. Preferably, the polypeptide has a molecular weight as determined by SDS-PAGE under reducing conditions of about 180 kD, about 170 kD, about 160 kD or about 120 kD.

The polypeptide of this aspect of the invention may comprise the amino acid sequence SEQ ID NO. 2 or SEQ ID NO. 3. Polypeptides are also disclosed comprising the amino acid sequence SEQ ID NO. 4, SEQ ID NO. 5, or an amino acid sequence having at least 80%, at least 90%, or at least 95% identity to the amino acid sequence of any of SEQ ID NO.1, SEQ ID NO. 2, SEQ ID NO. 3, SEQ ID NO. 4 or SEQ ID NO. 5.

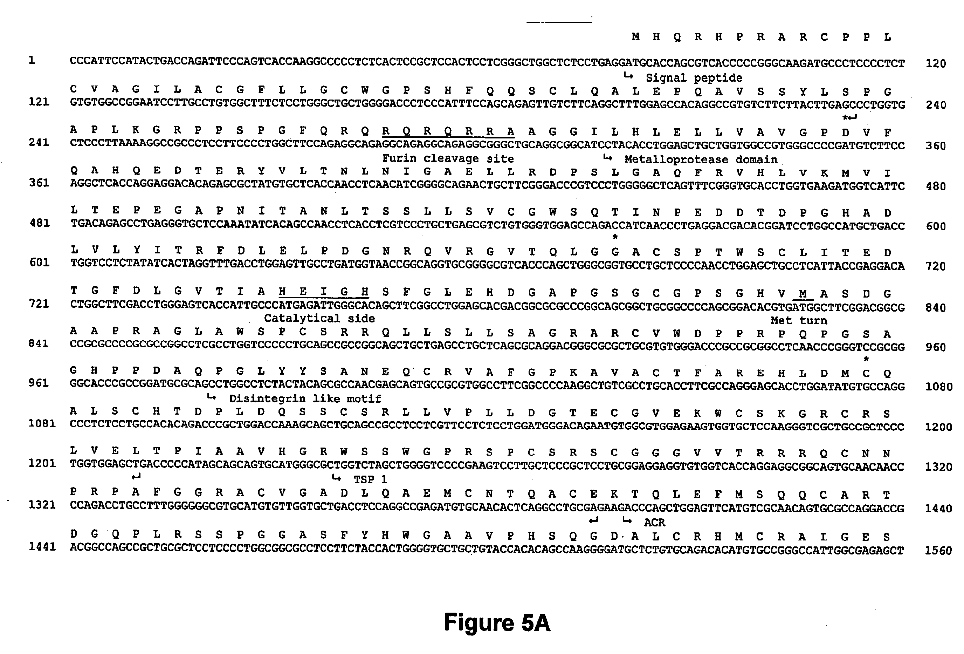

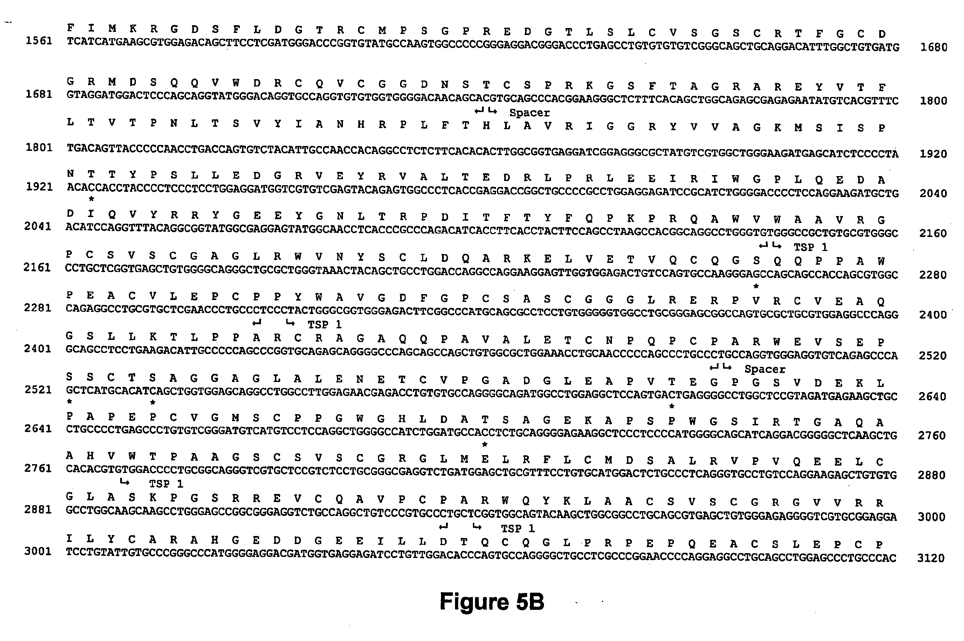

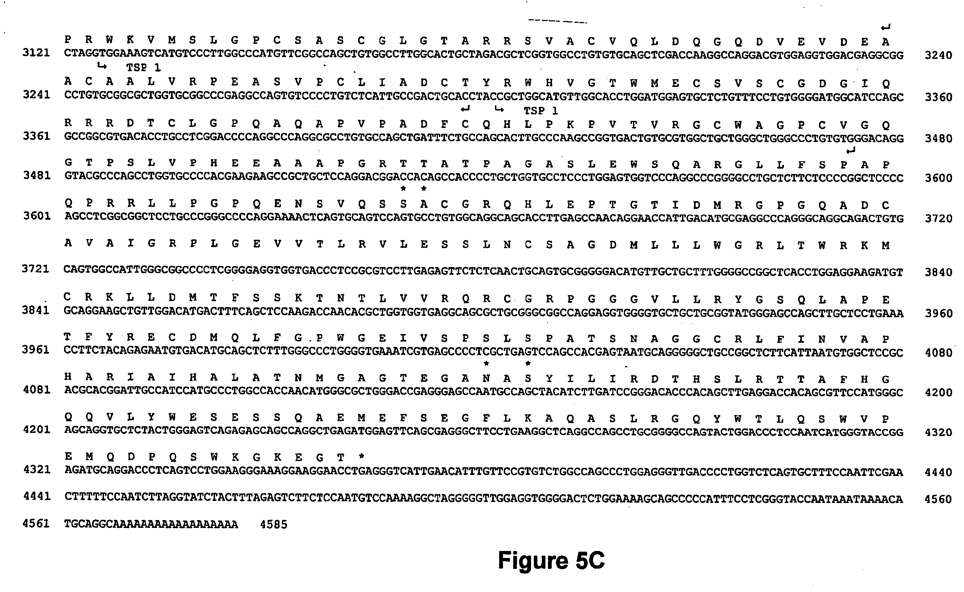

Also disclosed is a polypeptide which has an amino acid sequence as shown in Fig. 5 and a partial sequence thereof of at least 12, or at least 15 amino acids. The vWF-cp polypeptide of the invention retains vWF cleaving protease activity in the presence of the serine protease inhibitor diisopropyl fluorophosphate and in the presence of the calpain inhibitor Z-Leu-Leu-Tyr-CHN2.

The term "polypeptide" means a chain of amino acid residues linked through peptide bonds between the α-carboxyl carbon of one amino acid residue and the α-nitrogen of the next amino acid, and comprising 10 or more amino acid residues joint together. The polypeptide may contain other than the 20 gene-encoded amino acids. "Polypeptide" refers to both short chains, such as peptides, oligopeptides, or oliogomers, and to longer chains, generally referred to as proteins. "Polypeptide" include amino acid sequences modified either by natural processes, such as posttranslational modifications, or chemical modifications, which are known in the art. Modifications can occur anywhere in the polypeptide. The modification may lead to a variant of the polypeptide which may differ in amino acid sequence from original polypeptide by one or more substitutions, additions, deletions, fusions or any combination, or a naturally occurring variant, such as an allelic variant or non-naturally occurring variant made by mutagenesis or genetic engineering. The "Polypeptide" may be in form of an unprocessed or partial processed precursor, the "mature" polypeptide, or a fragment having an amino acid sequence that entirely is the same as a part, but not all, of the amino acid sequence of the longer a polypeptide chain. Fragment may be a shorter single continuous region of the longer chain, or comprised within a larger polypeptide of which they form a part. A Fragment of polypeptide may include, for example a truncated polypeptide having a partial amino acid sequence of the vWF-cp. A "Fragment" can be a, biological active fragment that mediate vWF cleaving protease activity, including those with similar activity or improved activity, or with decreased activity.

"Identity" means the identity of the amino acid sequence of a polypeptide to the amino acid sequence of a polypeptide comprising the sequence of SEQ ID. NO 1, SEQ ID NO. 2, SEQ ID NO. 3, SEQ ID NO. 4 or SEQ ID NO. 5. "At least 80% identity", means that the amino acid sequence is identical except that the polypeptide sequence differs in not less than 20 amino acids per 100 amino acids. "At least 90%" or "at least 95% identity" means a difference of not less than 10, or not less than 5 amino acids per 100 amino acids.

The term "vWF cleaving protease" (vWF-cp) means a protein or polypeptide having vWF cleaving activity and cleaves vWF high molecular vWF multimers in molecules of lower molecular weight which still have vWF activity and properties.

The term "vWF cleaving protease polypeptide" means a polypeptide having at least 10 amino acid residues. The complete amino acid sequence of the unprocessed polypeptide comprises 1427 amino acids. The "mature" polypeptide, which is a part of the larger unprocessed polypeptide should normally begin with or near amino acid 75, beginning with AAGGILH, and continuing to the carboxyl terminus. The mature vWF cleaving protease has a calculated polypeptide mass of about 145 KD. Additional amino acids which contains secretory or leader sequences, pro-sequences, sequences which aid purification or identification such as multiple His residues, a FLAG tag or and additional sequence for stability during recombinant production, can be included.

The term "vWF cleaving activity" means a physiological vWF cleaving activity which is defined by (1) the cleaving vWF at the peptide bond 842Tyr-843Met, (2) having a direct proteolytic activity which converts vWF having a singlet structure to vWF having a satellite structure, and (3) retaining activity in the presence of a serine protease inhibitor such as diisopropyl fluorophosphate (DFP) and in the presence of a calpain protease inhibitor such as carbobenzyloxy (Z) peptidyl diazomethylketone inhibitor (Z-Leu-Leu-Tyr-CHN₂). The proteolytic entities provided with the present invention may also act indirectly via another effector protein, for example a protease. This polypeptide can form an active vWF cleaving complex together with a metal ion selected from the group consisting Ca⁺⁺, Sr⁺⁺, Mg⁺⁺ and Ba⁺⁺. The preferred metal ion is Ca⁺⁺. This active complex is able to cleave vWF in a physiological manner as described above. The vWF cleaving activity of the polypeptide according to the present invention as defined in Claim 1 may be determined by any method described in the art, such as the method according to Furlan et al. (1996, Blood 87: 4223-4234).

The polypeptide of the invention may be isolated.

The term isolated" means altered from the natural state and/or removed from its original environment.

The polypeptide of the invention may be substantially pure.

The term "substantially pure" means purity as high as the relative proportions of polypeptide chains with the vWF-cp activity present in an amount of above 50%, especially above 80%, most preferred about 90%, of total protein compared to the vWF protease activity in plasma. The purity is determined by a purity as determined by SDS-PAGE (silver stained or Commassie stained) and Western blot and a ratio of vWF protease polypeptide to total protein amount, said purity is preferably greater than about 98%. If the purified vWF-cp polypeptide is purified from plasma the preparation contains between 0.001% and 1%, preferably 0.002% of the initial amount of plasma protein, and at least 1%, preferably at least 2.3% of the initial enzyme activity, which may be partially inactivated during the purification procedure. The preparation comprising a vWF - cp polypeptide according to the present invention is essentially free of vWF or vWF fragments, i.e. having a vWF content of below 5%, preferably below the detection limit of an assay used to detect vWF, such as a collagen-assay described in EP 0 816 852.

The substantially pure vWF-cp polypeptide may exhibit an apparent molecular weight of about 180 KD, 170 kD, 160 kD or 120 KD in SDS-PAGE analysis under reducing conditions.

It is also disclosed that the substantially pure vWF protease polypeptide may exhibit an apparent molecular weight of about 150 KD, 140 kD, 130 kD or 110 KD in SDS-PAGE analysis under non-reducing conditions.

The SDS-PAGE is performed under reducing conditions or non-reducing conditions. It is well known in the art, that molecular weight determination using SDS PAGE results in the detection of apparent molecular masses, which may be different from the molecular masses of the native, non-denatured protein.

Analysis of non-denatured material by mass spectrometry showed very broad peaks of high molecular weight. This finding is in agreement with appearance, in gel filtration experiments, suggesting that the proteins in this preparation tend to polymerize under physiologic conditions (a property of clusterin).

The polypeptide of the invention can be isolated from any source which comprises a vWF-cp polypeptide.

The term "source" means human plasma, a supernatant of a cell culture expressing a vWF -cp polypeptide according to the present invention, milk or other body fluids of transgenic animals expressing the polypeptide of the invention.

Purification of vWF-cp polypeptide can be performed by a combination of chromatographic steps including immunoaffinity chromatography, gel filtration, and ion exchange chromatography. For example, the first purification step can be immunoaffinity chromatography, the second step can be gel filtration, followed by one or more additional immunoaffinity chromatography steps. A further purification can be performed using ion exchange chromatography, preferably anion exchange chromatography and at least one affinity chromatography. Further purification steps can be performed using ion exchange chromatography, gel filtration and further affinity chromatography steps.

According to a second aspect of the invention there is provided a composition comprising a vWF-cp polypeptide of the first aspect of the invention. Also provided is a composition comprising a polypeptide comprising an amino acid sequence selected from the group of SEQ ID NO. 4 and SEQ ID NO. 5, and an amino acid sequence having at least 80% identity to the amino acid sequence of any of SEQ ID NO. 1, SEQ ID NO. 2, SEQ ID NO. 3, SEQ ID NO. 4 or SEQ ID NO. 5.

The composition of the invention comprising the polypeptide may further comprise a divalent metal ion selected from the group of Ca²⁺, Sir²⁺, Ba²⁺ and Mg²⁺.

A further aspect of the present invention relates to a complex of isolated vWF-cp polypeptide having vWF-cp activity, vWF and a metal ion selected from the group consisting of Ca²⁺, Sr²⁺, Ba²⁺ and Mg²⁺. The preparation may comprise divalent metal ions in a concentration of about 1 to 10⁶ ions per polypeptide molecule with vWF protease activity and can contain vWF -cp polypeptide in an essentially purified form.

The composition may comprise clusterin or an analog or derivative thereof having clusterin activity. With relation to the activity of the protein, the term "derivative" or "analog" of clusterin refers to a polypeptide that show the same characteristics as the native clusterin protein.

Clusterin is a heterodimeric glycoprotein consisting of two non-identical subunits, with a molecular mass of approximately 80 kDa (Rosenberg et al. 1995, Int. J. Biochem. Cell Biol. 27: 633-645; Tschopp et al. 1994, Clin. Exp. Immunol. 97(Suppl. 2): 11-14). It is produced in a wide array of tissues and found in most biologic fluids. The physiologic functions described in the prior art include complement regulation, lipid transport, sperm maturation, initiation of apoptosis, endocrine secretion, membrane protection and promotion of cell interactions. It has been found that the unusually high stability of the vWF -cp polypeptide of the present invention in circulating plasma is associated with the presence of clusterin and that the half-life of vWF cleaving protease activity *in vivo* is between 1 and 4 days, while other proteases in plasma have half-lives in the range of seconds to hours. The ratio of clusterin to vWF protease polypeptide in a composition according to the present invention is preferably in a range of 10M:1M to 1M:10M, and more preferably the ratio of clusterin and vWF is in the equimolar range. In human plasma, the concentration of vWF-cleaving protease is 2 - 10 mg/liter whereas that of clusterin is 50 - 400 mg/liter plasma (the molar ratio of vWF-cleaving protease to clusterin in human plasma is about 1:20 - 1:100).

According to a third aspect of the invention there is provided a nucleic acid molecule comprising a nucleic acid sequence encoding the polypeptide of the first aspect of the invention. Also provided is a nucleic acid molecule comprising a nucleic acid sequence encoding a polypeptide having an amino acid sequence selected from the group of SEQ ID NO. 4 and SEQ ID NO. 5, and an amino acid sequence having at least 80% identity to the amino acid sequence of any of SEQ ID NO. 1, SEQ ID NO. 2, SEQ ID NO. 3, SEQ ID NO. 4 or SEQ ID NO. 5.

The term "nucleic acid molecule" means a polynucleotide and generally refers to any DNA or RNA, which also includes variants of the DNA or RNA, the DNA or RNA which may be modified or unmodified, single- and double stranded or a mixture thereof, or a short polynucleotide, generally referred to as oligonucleotides. A typical variant of a polynucleotide differs in nucleotide sequence from the original polynucleotide in sequence and may or may not alter the amino acid sequence of the polypeptide encoded by the reference polynucleotide. Nucleotide changes may result in amino acid substitutions, additions, deletions, fusions or truncations in the polypeptide sequence of the polypeptide. The polynucleotide sequence may be modified to improve recombinant expression. Such modification includes use of highly translated codons. Nucleic acid sequence which do hybridize with the DNA sequence as shown in Fig. 5 or partial sequence encoding an amino acid of at least 12 amino acid are within the scope of the present invention. Hybridization Techniques are well known to the skilled artisan.

According to a fourth aspect of the invention there is provided an expression vector comprising a nucleic acid molecule of the third aspect of the invention. Also provided is an expression vector comprising a nucleic acid molecule comprising a nucleic acid sequence encoding a polypeptide having an amino acid sequence selected from the group of SEQ ID NO. 4 and SEQ ID NO. 5, and an amino acid sequence having at least 80% identity to the amino acid sequence of any of SEQ ID NO. 1, SEQ ID NO. 2, SEQ ID NO. 3, SEQ ID NO. 4 and SEQ ID NO. 5.

The nucleic acid molecule of the invention can be used for constructing expression systems providing appropriate elements for replication of vector within a host cells and expression of the DNA which can then be used for the expression of a polypeptide having vWF cleaving protease activity according to the present invention. The nucleic acid sequence may be modified to improve expression, such as addition of a Kozak sequence.

The expression vector may comprise, for example, in the direction of transcription, a transcriptional regulatory region and a translational initiation region functional in a host cell, a DNA sequence comprising a polynucleotide expressing a VWF -cp polypeptide according to the present invention and translational and transcriptional termination regions functional in said host cell, wherein expression of the nucleic sequence is regulated by the initiation and termination regions. The expression vector may also contain elements for the replication of the nucleotide. Examples of DNA expression vectors are pBPV, pSVL, pRc/CMV, pRc/RSV, myogenic vector systems, pcDNA3 based vector (Invitrogen) or vectors derived from viral systems, for example from vaccinia virus, adenoviruses, adeno-associated virus, herpes viruses, retroviruses or baculo viruses. Suitable mammalian expression vectors usually contain one or more eukaryotic transcription units that are capable of expression in mammalian cells. The transcription unit is comprised of at least a promoter element to mediate transcription of foreign DNA sequences. Suitable promoters for mammalian cells are known in the art and include viral promoters such as that from simian virus 40 (SV40), cytomegalovirus (CMV), Rous sarcoma virus (RSV), adenovirus (ADV), bovine papilloma virus (BPV) or other promoters selected from the group of methallothionein promoter and ß-Actin promoter. Other promoters known in the art are also applicable for expression.

The expression vector containing the nucleic acid sequence which encodes the vWF-cp polypeptide according to the present invention can be used to transform host cells which then produce the polypeptide. The transformed host cells can be grown in a cell culture system to produce the polypeptide *in vitro*. The host cells can excrete the polypeptide having vWF protease activity into the cell culture medium from which it can be prepared or the polypeptide can be isolated from the cell lysate.

According to a fifth aspect of the invention there is provided a host cell comprising an expression vector of the fourth aspect of the invention.

Also provided is a host cell comprising an expression vector comprising a nucleic acid molecule comprising a nucleic acid sequence encoding a polypeptide comprising an amino acid sequence selected from the group of SEQ ID NO. 4 and SEQ ID NO. 5, and an amino acid sequence having at least 80% identity to the amino acid sequence of any of SEQ ID NO. 1, SEQ ID NO. 2, SEQ ID NO. 3, SEQ ID NO. 4 or SEQ ID NO. 5. The expression vector can be transformed or transfected into a host cells for expression of the recombinant polypeptide. Suitable host cells include any cell capable of producing vWF -cp polypeptide after being transformed or transfected. Preferred cells include bacterial cells, yeast, insect cells or animal cells. The host cell may be a cell derived from the body of a mammal, for example fibroblasts, keratinocytes, hematopoietic cells, hepatocytes or myoblasts, which are transformed *in vitro* with an expression vector system carrying a nucleic acid according to the present invention and re-implanted into the mammal. The polypeptide according to the present invention encoded by said nucleic acid will be synthesized by these cells *in vivo* and they will exhibit a desired biological activity in the mammal.

Mammalian cell lines available as hosts for expression are known in the art and include many immortalized cell lines available from the American Type Culture Collection (ATCC). Exemplary mammalian host cells include particularly primate cell lines and rodent cell lines, including transformed cell lines. Preferably for stable integration of the vector DNA, and for subsequent amplification of the integrated vector DNA, both by conventional methods, Chinese hamster ovary (CHO) cells are employed as a mammalian host cell of choice. Other suitable cell lines include, but are not limited to, HeLa cells, baby hamster kidney (BHK) cells, monkey kidney cells (COS-1), human hepatocellular carcinoma cells (e.g., Hep G2), human adenovirus transformed 293 cells, HEK 293cells, SKHep cells, mouse L-929 cells, HaK hamster cell lines, murine 3T3 cells derived from Swiss, Balb-c or NIH mice and a number of other cell lines. Another suitable mammalian cell line is the CV-1 cell line. Normal diploid cells, cell strains derived from in vitro culture of primary tissue, as well as primary explants, are also suitable. Candidate cells may be genotypically deficient in the selection gene, or may contain a dominantly acting selection gene.

Host cells can be either transformed cells or untransformed cells. The host cells are preferably those expressing furin either naturally or after being genetically engineered to express recombinant furin.

The selection of suitable mammalian host cells and methods for transformation, culture, amplification, screening and product production and purification are known in the art.

The host cell transformed with a vector carrying the vWF-cp polypeptide en coding nucleic acid may then be cultured under suitable conditions if desired, with amplification introduced genes. Effective conditions include, but are not limited to, appropriate media, bioreactor, temperature, pH and oxygen conditions that permit protein production. The method of this present invention therefore comprises culturing a suitable cell or cell line, which has been transformed with a nucleic acid sequence encoding a amino acid sequence of the vWF-cp polypeptide, the coding sequence under the control of a transcriptional regulatory sequence. The expressed polypeptide is then recovered, isolated and purified from the culture medium if secreted by the cells or from the cell, if expressed intracellularly by appropriate means known to one of skill in the art.

The nucleic acid molecule of the invention or a fragment thereof can be expressed in a eukaryotic or prokaryotic microorganism system, such as fungi, including yeast, or bacteria. Fragments can include truncated forms of the vWF cleaving protease. Examples of truncation include, but are not limited to, N-or C-terminal deletions.

The nucleic acid molecule of invention may also be used to generate transgenic animals, which express said polypeptide *in vivo.* In one embodiment of this specific application, the transgenic animals may express the vWF -cp polypeptide in endogenous glands, for example in mammary glands from which the said proteins are secreted. In the case of the mammary glands, said vWF protease polypeptide is secreted into the milk of the animals from which said proteins can be prepared. The animals may be mice, cattle, pigs, goats, sheep, rabbits or any other economically useful animal.

The wWF-cp polypeptide of the invention can be used in a method of purification of vWF by providing as a ligand for vWF a vWF-cp polypeptide of the invention, contacting a solution comprising vWF with the vWF-cp polypeptide ligand under conditions whereby vWF is bound to the ligand and recover from said ligand purified vWF. The vWF-cp polypeptide ligand may be bound to a solid carrier.

A further aspect of the invention is a method for production of anti-vWF-cp polypeptide antibodies by immunization of an animal with a polypeptide of the invention and isolation of anti-vWF-cp polypeptide antibodies from the animal.

It is disclosed that the vWF-cp polypeptide of the invention can also be used for the development of other vWF-cp polypeptide binding molecules using techniques known in the art.

"Antibodies" as used herein includes polyclonal and monoclonal antibodies, chimeric antibodies, single chain, and humanized antibodies, as well as Fab fragments, including the products of an Fab or other immunoglobulin expression library, peptides or peptidomimetics. Monoclonal antibodies can be produced according to methods well known in the art. Other binding molecules can be derivatives of a antibody, such as a single chain antibody, a Fab-, or Fab' 2-fragment, a chimeric antibody, or a peptide or a peptidomimetic, which can be obtained by known methods such as e.g. the phage display method.

It is also disclosed that a vWF-cp polypeptide binding molecule selected from the group of single chain antibodies, Fab- or Fab' 2-fragment or polypeptides with vWF protease binding site can be produced by a method, wherein a phage display library is screened for anti-vWF-cp polypeptide binding molecule, the nucleic acid sequence of positive clones is determined and a nucleic acid molecule comprising the sequence is cloned into an expression vector.

The development of the antibodies, antibody derivatives, peptidomimetics or any other molecule which binds vWF-cp polypeptide can be accomplished according to methods known to the prior art (Greer et al. 1994, J. Med. Chem. 37: 1035-1054; Kemp 1990, Trends Biotechnol. 8: 249-255).

Another aspect of the invention is a method for purification of vWF-cp polypeptide using a vWF-cp polypeptide binding molecule as a ligand to purify vWF-cp, as defined in Claim 17. Such a method can be performed by contacting a solution comprising vWF-cp polypeptide with the vWF-cp polypeptide binding molecule under conditions whereby vWF-cp polypeptide is bound to the binding molecule and recovering purified vWF-cp polypeptide by selectively eluting vWF-cp polypeptide from the vWF-cp binding molecule. The vWF-cp binding molecule may be bound to a solid carrier.

A further aspect of the invention is a method for detection of vWF-cp polypeptide in a sample using a vWF-cp polypeptide binding molecule as defined in claim 18. Thereby a solution suspected to contain vWF-cp polypeptide is contacted with a vWF-cp polypeptide binding molecule as described above under conditions allowing the formation of a complex of vWF-cp polypeptide/vWF-cp polypeptide binding molecule and detection of the complex.

The vWF-cp polypeptide of the invention can be used, for example, to process plasmatic or recombinantly produced vWF. Recombinant vWF (r-vWF) can be produced in CHO cells, e.g. according to Fischer et al. (1995. FEBS Lett 375: 259-262). The r-vWF recovered in this manner is available as a mature vWF and has a singlet structure, i.e. it differs from plasma-derived vWF, which always has a characteristic satellite structure when examined on 2% SDS agarose gels. US5,854,403 teaches that r-vWF is comprised of multimers with high structural integrity which is retained even after purification and treatment for the inactivation of viruses. The intact structure of the r-vWF is defined by a result of electrophoretic analysis consisting of multimer bands with an absence of satellite bands. To prepare an r-vWF preparation having a structure more closely corresponding to that of plasma-derived vWF from r-vWF with singlet structure, r-vWF is treated with the vWF protease polypeptide or a composition comprising the vWF-protease polypeptide of the present invention, and optionally metal ions and/or clusterin.

According to one aspect of the invention, the vWF-cp polypeptide can be used for the production of a preparation for the prophylaxis and therapy of diseases that show supranormal vWF content or an increased level of high-molecular weight vWF in patients, such a thromboembolic disease. This can result in thromboses and thromboembolic diseases. For example, thrombotic throbocytic purpura (TTP), Henoch-Schönlein purpura, preeclampsia, neonatal thrombocytopenia or haemolytic-uremic syndrome. By administering an effective dose of a polypeptide of the invention and having a vWF protease activity, this can lead to reduction of the content of high molecular weight vWF multimers in the patients, resulting in effective therapy of these diseases. The disease can be selected from the thromboembolic disease is thrombotic thrombocytic purpura (TTP), Henoch-Schönlein purpura, preeclampsia, neonatal thrombocytopenia or hemolyticuremic syndrome.

The invention is described in the following examples, without being limited thereto.

### Examples

### Example 1:

### Isolation of vWF-cp polypeptides

1.1. Preparation of an IgG-eTTP-coupled affinity gel. The IgG-eTTP is isolated by aid of a 20 ml protein A-Sepharose® (diameter 1.6 cm) in TBS, pH 7.4. Pheresis plasma of a patient suffering from acquired TTP ("erworbenes" TTP; eTTP), which previously had been assayed for its inhibitor content relative to the vWF-cleaving protease is applied to the column in a volume of 50 m1. After subsequent rinsing with TBS, pH 7.4, the bound IgGs are step-wise eluted with citrate, 0.1 M, pH 4.0, and glycine, 0.1 M, pH 2.7. The fractions immediately are brought to a physiological pH by adding Tris, 1.5 M, pH 8.8, and dialysed against TBS, pH 7.4. The Affi-Gel® Hz is coupled according to the producer's instructions with the IgG-eTTP which had been ished out of the protein A-Sepharose® with a pH of 4.0. The column material prepared in this manner first is ished as prescribed, subsequently it is washed 3 times alternatingly with 50 ml of buffer B and 200 ml of buffer A (chapter 1.7). Prior to use, intensive rinsing with buffer A is carried out in each instance.

### 1.2 First Step

As the starting material, 100 ml of pooled CPD plasma which had come from at least three donors and had been stored at -20°C, is used after centrifuging at 2,500 rpm (1,100g) for 5 min. At a relatively low flow rate (FR: 30 ml/h), the plasma is loaded on a 200 ml chromatographic column with IgG-eTTP Affi-Gel® Hz (hydrazide, diameter 2.6 cm) which had been equilibrated in buffer A. After washing with at least 400 ml of buffer A over night at the same flow rate, a 200 ml desalting gel filtration column (Bio-Gel® P-6DG, diameter 2.6 cm) and a 10 ml protein G-Sepharose® (diameter 1.6 cm), which previously also had been rinsed with buffer A, is connected thereto. After the flow rate had been increased to 100 ml/h, the proteins bound to Affi-Gel Hz are eluted directly with 50 ml of buffer B onto the Bio-Gel® P-6DG so as to remove from the proteins the NaSCN that had been in buffer B. The proteins which had been eluted from the desalting column prior to the NaSCN are led through the protein G Sepharose® without interruption, where they are freed from the IgGs. Here, the flow rate is loared to 50 ml/h so as to extend the dwell time of the proteins in the 10 ml column. For regeneration, the protein G-Sepharose® is shortly washed with buffer C, and the eluted IgG fraction is stored for analysis.

The first step is carried out 8 times before the collected fractions which had been frozen at -20°C are pooled and further processed.

### 1.3 Second Step

The pooled fractions from 8 chromatographies of the first step are diluted 1:1 with H₂O so as to obtain an ionic strength at which the desired proteins would bind to the anion exchange column (High Q Support®). The sample whose volume is from 1,500 to 1,800 ml, depending on the charge used, is checked for its pH and its ionic strength and applied over night at a FR of 90 ml/h through a 50 ml column with Therasorb® (diameter 1.6 cm) onto a 5 ml High Q Support® (diameter 1.6 cm). Both, Therasorb and High Q Support® had previously been equilibrated in buffer D. After washing with approximately 150 ml of buffer D, the Therasorb is disconnected, and the 25 ml Lentil Lectin Sepharose® (diameter 1.6 cm) which had been equilibrated in buffer E is connected to follow the High Q Support®. At a FR of 60 ml/h, the proteins bound to High Q Support are immediately eluted with buffer E directly to the Lentil Lectin-Sepharose®. The proteins which bound to the Lentil Lectin-Sepharose® can be eluted in two steps with buffers G and H and canbe collected. The proteins which had remained bound to Therasorb and High Q Support® are washed out with buffer C or buffer F, respectively, and discarded after an analysis.

For regeneration, before being used, the Lentil Lectin-Sepharose® in each case is rinsed according to the producer's instruction 3 times alternatingly with 20 ml each of buffers I and J, the High Q Support is rinsed successively with 10 ml each of NaOH IN and NaCl 1M.

### 1.4 Third Step

The pooled fractions which had been eluted from the Lentil Lectin-Sepharose® with buffer H are dialysed three times for a total of 4 h, each against 1 l of buffer D, and again applied to the High Q Support at a flow rate of 60 ml/h. Connected thereinfront is a 5 ml heparin-Sepharose (diameter 1.4 cm), which likewise had been equilibrated in buffer D. After the application of the sample, it is rinsed with approximately 50 ml of buffer D, the heparin-Sepharose is disconnected, and a 500 ml Sephacryl® S-300 HR (diameter 2.6 cm), which had been equilibrated in buffer L, is connected thereto. The proteins bound to High Q Support are directly eluted to the gel filtration column with 10 ml of buffer K. The exclusion chromatography is effected at a flow rate of 42 ml/h, and the fractions are collected at 7 ml each. The proteins which are more strongly bound to High Q Support@ are again eluted with buffer F, those which remained adhered to the heparin-Sepharose, with buffer K.

### 1.5 Fourth Step

The pool of the active fractions from the third step is applied without treatment at a FR of 10 ml/h to a 1 ml anti-α₂-macroglobulin column (flow rate0.7 cm) which had been equilibrated in buffer L. The anti-α₂-macroglobulin column is prepared by immobilization according to the instructions, of rabbit-anti-α₂-macroglobulin antibodies at a concentration of 4.9 mg/ml on CNBr-activated Sepharose. The proteins bound thereon are eluted with NaSCN 3M in buffer L and with buffer C and stored for analysis.

**Table 1: List of buffers**

| | | | |
|---|---|---|---|
| buffer A | Tris | 10 mm | pH 7.4 |
| | NaCl | 0.15 M | |
| | Na₃-citrate | 1mM | |
| | Na acid | 0.02% | |
| buffer B | NaSCN | 3.0M | pH 7.4 |
| | in buffer A | | |
| buffer C | glycine | 0.1M | pH 2.7 |
| | Na acid | 0.02% | |
| buffer D | Tris | 10mM | pH 7.4 |
| | NaCl | 75mM | |
| buffer E | Tris | 20mM | pH 7.4 |
| | NaCl | 0.5M | |
| | MnCl₂ | 1 mM | |
| buffer F | Tris | 10mM | pH 7.4 |
| | NaCl | 1.0M | |
| buffer G | Tris | 20mM | pH 7.4 |
| | NaCl | 0.5M | |
| | Methyl-α-D-mannopyranoside | 30mM | |
| buffer H | Tris | 20mM | pH 7.4 |
| | NaCl | 0.5M | |
| | Methyl-α-D-mannopyranoside | 0.3M | |
| buffer I | Tris | 20mM | pH 8.5 |
| | NaCl | 0.5M | |
| buffer J | Na acetate | 20mM | pH 5.5 |
| | NaCl | 0.5M | |
| buffer K | Tris | 10mM | pH 7.4 |
| | NaCl | 0.5M | |
| buffer L (TBS) | Tris | 10mM | pH 7.4 |
| | NaCl | 0.15M | |

**Table 2: List of Chromatographic materials**

| **Material** | **Provider /Company** |
|---|---|
| affi-gel hydrazide gel®: for immobilizing specific IgG's | Bio-Rad, Hercules CA USA |
| anti-α₂-macroglobulin column: isolation of α₂-macroglobulin | applicant's own production (see 1.5): rabbit-anti-human-α2-macroglobulin antibody on CNBr activated Sepharose 4B; 4.9 mg/ml |
| Bio-Gel® P6-DG, medium: gel filtration with exclusion limit > 6kDa | Bio-Rad |
| CNBr activated Sepharose 4B®: for immobilizing proteins | Amersham Pharmacia Biotech, Uppsala, S |
| heparin Sepharose, HITrap® 5ml: affinity chromatography: binds various proteins | Amersham Parmacia |
| High Q Support®, Macro-Prep: strong anion exchanger | Bio-Rad |
| IgG-eTTP Affi-gel Hz: for binding vWF-protease | applicant's own production (see 1.1.): IgG-eTTP on Affi-gel Hz hydrazide |
| Lentil Lectin-Sepharose 4B: affinity chromatography: binds to sugar residues of proteins | Amersham Pharmacia |
| protein A Sepharose® CL-4B. binds IgG of type 1, 2 and 4 | Amersham Pharmacia |
| protein G Sepharose® 4FF: isolation of IgGs of all types | Amersham Pharmacia |
| Sephacryl® S-300 HR: gel filtration for MWs 10,000 to 1,500,000 | Amersham Pharmacia |
| Therasorb: coupled with sheep-anti-human-Ig-antibodies: isolation of human immunoglobulins | Serag-Wiessner, Naila, D |

### 1.6 Fifth Step

Alternatively, or in addition to step four, an anti-clusterin column chromatography as a further step can be applied. The samples are prepared identically to the anti-α2- macrogluobulin-column using anti-clusterin antibodies.

### 1.7. SDS-Page reduced/non-reduced

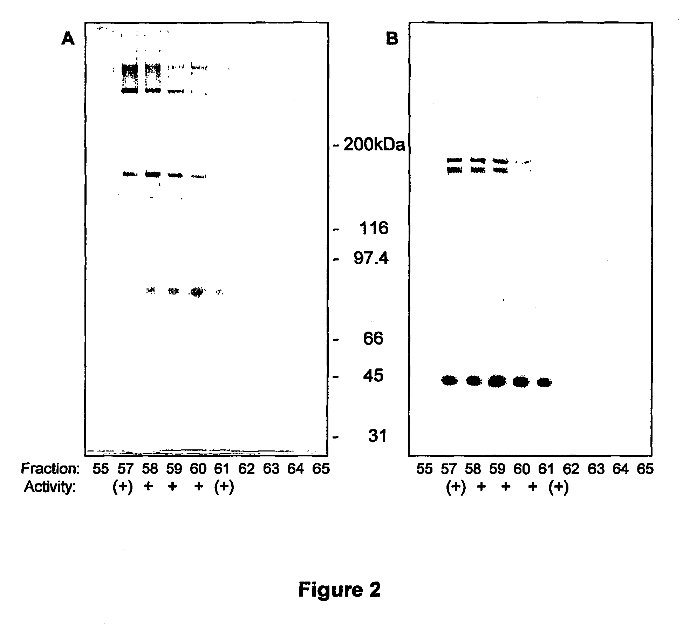

The final preparation from the third step of isolation is electrophoresed on a 1.5 mm-thick SDS-polyacrylamide gel according to Laemmli (1970, Nature, 227: 680-685). A gradient of 4 to 12% polyacrylamide is used for fractionation of proteins. After electrophoresis under non-reducing or reducing conditions (final concentration 65 mmol/l dithiotreitol DTT), the proteins are made visible by silver stain or Commassie blue stain (Fig. 2). Under non-reducing conditions bands having a molecular weight (MW) of about 150 kD, 140 KD, 130 KD and 110 KD are detectable and under reducing conditions of about 180 KD, 170 KD, 160 kD and 120 KD.

### 1.8 Determination of vWF protease activity

The different fractions isolated by the purification process as described above are tested for vWF cleaving activity by the method as described by Furlan et al. (1996. Blood 87: 4223-4234).

### Example 2:

### Amino acid sequencing and amino acid analysis of the vWF-cp polypeptide

The final protein preparation from the third step of isolation is electrophoresed on a 1.5 mm-thick SDS-polyacrylamide gel according to Laemmli (1970, Nature, 227: 680-685). A gradient of 4 to 12% polyacrylamide is used for fractionation of high molecular weight proteins, and a gradient of 8 to 12% polyacrylamide for low molecular weight proteins. After electrophoresis under non-reducing or reducing conditions (final concentration 65 mmol/l dithiotreitol), the proteins are blotted onto PVDF-membranes and stained for 2 min with 0.25% Coomassie Blue in 45% methanol, 9% acetic acid and 46% H₂O. After rinsing with a mixture of 50% methanol, 10% acetic acid and 40% H₂O, the visible protein bands are cut out and analyzed on a Procise-cLC Sequencer (Foster City, CA) at the Chemical Institute of the University of Bern.
The N-terminal amino acid sequence of polypeptide bands separated by SDS-PAGE of purified vWF-cleaving protease is shown in Table 3.

**Table 3: Determination of N-terminal amino acid sequence of vWF-cp**

| **Molecular weight** | **Amino Acid sequence** |
|---|---|
| 350 kDa unreduced | |
| 150 kDa unred. | Ala-Ala-Gly-Gly-Ile |
| 140 kDa unred. | Ala-Ala-Gly-Gly-Ile |
| 130 kDa unred. | Ala-Ala-Gly-Gly-Ile |
| 110 kDa unred. | Ala-Ala-Gly-Gly-Ile-Leu-His-Leu-Glu |
| 70 kDa unred. | |
| 180 kDa reduced | Ala-Ala-Gly-Gly-Ile-Leu-His-Leu-Glu |
| 170 kDa reduced | Ala-Ala-Gly-Gly-Ile |
| 160 kDa reduced | |
| 120 kDa reduced | |
| 40 kDa reduced | Asp-Gln-Thr-Val-Ser** |

| | |
|---|---|
| * identified as α2-macroglobulin ** identified as clusterin | |

Analysis of the composition of the amino acids is performed from the same sample as used for amino acid sequencing. The protein bands are hydrolyzed in the gas phase over 6 N HCl for 22 hours at 110°C and the amino acids are determined by high-performance liquid chromatography. Four unreduced polypeptide bands from SDS-PAGE of purified vWF-cp with Mᵣ 150, 140, 130, and 110 kDa are analyzed. The results are shown in Tab. 4.

**Table 4: Amino acid composition of isolated vWF-cp polypeptide**

| **Amino acid** | **No. residues/100 residues** | | | |
|---|---|---|---|---|
| | 150 kDa | 140 kDa | 130 kDa | 110 kDa |
| Asx | 6.7 | 7.0 | 7.4 | 8.3 |
| Glx | 12.2 | 12.0 | 12.8 | 11.8 |
| Ser | 8.4 | 8.9 | 8.8 | 9.2 |
| Gly | 11.8 | 12.1 | 12.1 | 12.9 |
| His | 2.5 | 2.3 | 2.5 | 2.4 |
| Arg | 8.3 | 7.6 | 8.1 | 7.2 |
| Thr | 5.6 | 5.5 | 5.6 | 5.7 |
| Ala | 10.1 | 9.5 | 9.6 | 8.2 |
| Pro | 8.9 | 8.5 | 8.3 | 8.1 |
| Tyr | 2.1 | 2.7 | 2.3 | 2.4 |
| Val | 7.0 | 6.9 | 6.7 | 6.5 |
| Ile | 2.7 | 2.9 | 2.6 | 3.0 |
| Leu | 10.0 | 9.9 | 9.1 | 9.7 |
| Phe | 2.6 | 2.8 | 2.6 | 3.2 |
| Lys | 1.0 | 1.3 | 1.3 | 1.4 |

### Example 3:

### Identification of the vWF-cp gene

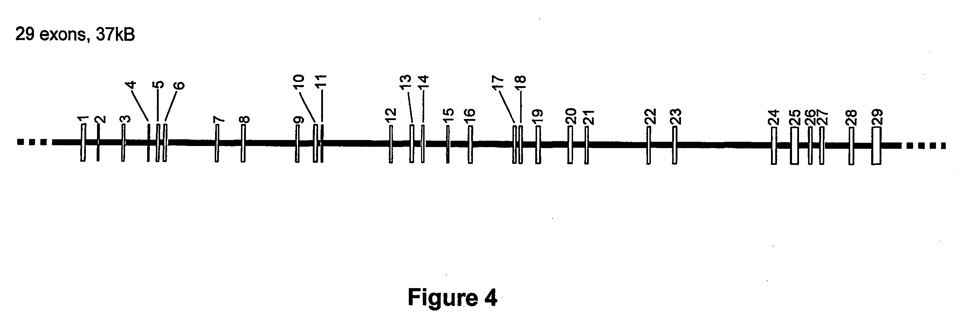

The nucleic acid coding sequence of the amino acid sequence of peptide AAGGILHLELLVAVG (SEQ. ID. NO.2) of the N-terminal 15 residues of the purified plasmatic human vWF-cp is determined and corresponds to the nucleic acid sequence GCT GCA GGC GGC ATC CTA CAC CTG GAG CTG CTG GTG GCC GTG GGC (SEQ. ID. No. 9). A database searching in the NCBI GenBank (http://www.ncbi.nlm.nih.gov/) showed the location of the corresponding nucleic acid sequence GCT GCA GGC GGC ATC CTA CAC CTG GAG CTG CTG GTG GCC GTG GGC (SEQ.ID.No. 9) on chromosome 9 clone RP11-244N20 (AL158826, GI11544459). Thus the nucleotide sequence from base 150001 to 185911 is screened for potential exons. Consecutive overlapping genome-segments with various lengths (1500 bases-5000 bases) are analysed using search engines that are queried via the internet-explorer. The genomic sequence segments, its translations and the results of the search are managed using the *'Vectors NTI Suite1 v.5.2'* computer-program (Informax Inc., USA). The sequence of RP11-244N20 has a size of approximately 185 kb and the identified nucleic acid sequence starts at position 156.653. Use of an exon search program (Grail, http://compbio.ornl.gov/Grail-1.3) allowed identification of about 30 putative exons upstream and downstream of position 156.653. The first four exons of the search are translated into the corresponding amino acid sequence and these sequences are searched for homologies. This search revealed that each sequence displays high homology with the amino acid sequence of the family of human disintegrin and metalloproteinases with thrombospondin motifs (ADAM-TS).
In order to connect the putative exons poly-A -RNA from liver is reverse transcribed and the cDNA is then PCR amplified with primers specific for some of the putative exons (Table 5). The PCR products of primers 6142 (SEQ.ID.No.16)-6277 (SEQ.ID.No.17), 6142 (SEQ.ID.No.16)-6546 (SEQ.ID.No.18), 6351 (SEQ.ID.No. 19)-6546 (SEQ.ID.No. 18), 6278 (SEQ.ID.No. 21)-6407 (SEQ.ID.No. 20), 6346 (SEQ.ID.No. 23)-6395 (SEQ.ID.No. 24) and 6406 (SEQ.ID.No. 25)-6506 (SEQ.ID.No. 26) (derived from putative exons 1-6, 3-11, 6-11, 10-14, 13-16, 14-23, respectively) revealed bands of the expected size. After sequencing an open reading frame from exon 1 to exon 23 located near to the 3' end of clone RP11-244N20 can be identified. The 3' end of the cDNA sequence is examined by PCR amplification of the cDNA using forward primer 6548 (SEQ.ID.No. 27) of exon 22 and the specific MC18 sequence of the RT primer spdT. The resulting fragment of a size approximately to 1.8 kb is sequenced and after database search the 3' part of the new cDNA sequence can be located on region q34 (AC002325) on chromosome 9.
The unknown sequence of the gene at the 5' end is identified by 5' -RACE and sequencing of the resulting PCR product revealed two additional exons. None of the next five exons upstream of exon 1-2 can be connected by PCR to the already known cDNA sequence. All together 29 exons with a cDNA size of 4.6 kb are found. The vWF -cleaving protease gene spans approximately 37 kb, a schematic drawing of the localization of the exons is shown in Figure 4. The complete cDNA sequence is depicted in Figure 5.

**Table 5 Sequences of Primers Used for Reverse Transcription, PCR and Cloning**

| | | |
|---|---|---|
| spdT | 5'-GAGCAAATTCCTGTACTGAC (T)₃₀ NN-3' (SEQ.ID.No. 12) | production of cDNA |
| | | |
| MC18 | 5'-GAGCAAATTCCTGTACTGAC-3' (SEQ.ID.No. 13) | specific part of spdT |
| 6434 | 5'-GACCACGCGTATCGACGTCGAC-3' (SEQ.ID.No. 14) | anchor Primer |
| 6275 | 5'-CTCAGGGTTGATGGTCTGGCT-3' (SEQ.ID.No. 15) | exon 5 reversed |
| 6142 | 5'-CGGGCTGCAGGCGGGATCCTACACCTGGAG-3' (SEQ.ID.No. 16) | exon 3 forward |
| 6277 | 5'-AATGGTGACTCCCAGGTCGAG-3'. (SEQ.ID.No. 17) | exon 6 reversed |
| 6546 | 5'-TGGAGGTCAGCACCAACACA-3' (SEQ.ID.No. 18) | exon 11 reversed |
| 6351 | 5'-GAGTTGCCTGATGGTAACCG-3' (SEQ.ID.No. 19) | exon 6forward |
| 6407 | 5'-GAGCCCTTCCGTGGGCTGCA-3' (SEQ.ID.No. 20) | exon 14 reversed |
| 6278 | 5'-CGCTCCCTGGTGGAGCTGACC-3' (SEQ.ID.No. 21) | exon 10 forward |
| 6561 | 5'-CTCACCATCGTCCTCCCCAT-3' (SEQ.ID.No. 22) | exon 23 reversed |
| 6346 | 5'-ATCATGAAGCGTGGAGACAGC-3' (SEQ.ID.No. 23) | exon 13 forward |
| 6395 | 5'-CCTGGAGGGGTCCCCAGATG-3' (SEQ.ID.No. 24) | exon 16 reversed |
| 6406 | 5'-TGCAGCCCACGGAAGGGCTC-3' (SEQ.ID.No. 25) | exon 14 forward |
| 6506 | 5'-CAGGGCTCCAGGCTGCAGGC-3' (SEQ.ID.No. 26) | exon 23 reversed |
| 6548 | 5'-AGGAAGAGCTGTGTGGCCTG-3' (SEQ.ID.No. 27) | exon 22 forward |
| 6725 | 5'-GACGCGGCCCAGCCGGCCGCTGCAGGCGGCATCCTACAC-3' (SEQ.ID.No. 28) | exon 3 forward -*Sfi*I |
| 6276 | 5'-GGCCCTCGAGCGGTTCCTTCCTTTCCCTTCCAGG-3' (SEQ.ID.No. 29) | exon 29 reversed -XhoI |

### Determination of Similarities to Other Proteins

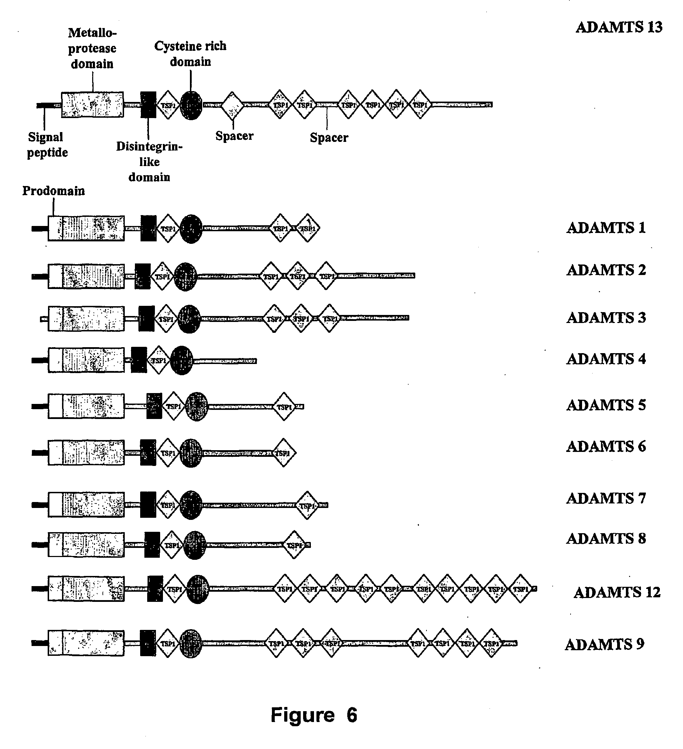

The SMART program (http://smart.eml-heidelberg.de) is searched for domain architectures of the protease and identified protein modules including a signal peptide with its transmembrane region, a furin cleavage site, a metalloprotease domain, i.e. a Zn -protease catalytic site consensus sequence (HEXXH), a Met turn, a disintegrin domain, a cysteine -rich region, and thrombospondin type 1 (TSP-1) motifs (Figure 6). These modules are also shared by members of the novel ADAMTS (for a disintegrin and metalloprotease with thrombospondin type 1 motif) metalloproteinase family (Tang 1999. FEBS Lett. 445:223-225; Tang 2001. Int. J. Biochem. Cell Biol. 33:33-44). The amino acid sequence of the vWF cleaving protease is aligned with all the human ADAMTS protein sequences, which are listed by the human genome organization (HUGO) gene nomenclature committee http://www.gene.ucl.ac.uk/nomenclature/genefamily/adamts.html A schematic representation of the domains is shown in Figure 6. In contrast to the ADAMTS proteins our putative new member of this family lacks a prodomain. However, examination of the amino acid sequences of the next five putative exons upstream of exon 1-2 showed no sequence homologies to ADAMTS proteins. In addition, no signal peptide sequences can be found on these exons. We thus named the vWF -cleaving protease ADAMTS13. The deduced amino acid sequence is also compared with the EMBL protein database (http://ww2.ebi.ac.uk). Exons 9 to 16 correspond partly to an already submitted hypothetical 39.9 kDa protein located on chromosome 9 and termed open reading frame 8 (C9ORF8, XM 005647, GI12735207). In addition exon 26 to 29 correspond to the hypothethical protein DKFZp434C2322 (NM032252, GI14149974) (Wiemann et al. 2001. Genome Res. 11: 422-435).

### Example 4:

### Affinity purification of von Willebrand Factor (vWF)

The peptide with the sequence AAGGILHLELLV (SEQ.ID.No. 1) is synthesized on a solid-phase support following the method of Barany et al. (1980. Solid-phase Peptide Synthesis. In: The Peptides. vol. 2 ,Gross, E. and Meienhofer, J. (eds.) Academic, New York, SEITEN). After cleavage and de-protection of the peptide, the peptide is purified by ion-exchange chromatography. The peptide is characterized by reverse phase HPLC on a C8 silica column with gradient elution in trifluoro acetic acid with acetonitrile. The peptide showed no major byproducts.

The peptide is solubilized in a concentration of 5 mg/mL in 0.1 molar phosphate buffer pH 7.5 and incubated with a pre-activated gel suitable for affinity chromatography (Actigel, ALD-Superflow, Sterogene). Prior to coupling of the peptide to the gel the pre-activated matrix is excessively washed with the same phosphate buffer. One volume of the pre-washed gel is then mixed with one volume of the peptide solution to be immobilized and subsequently 0.1 volume portions of a solution of 0.1 molar cyanoborohydride (NaCNBH₃) in 0.1 molar phosphate buffer pH 7.5. The gel is suspended in this solution and shaked for 15 hours at room temperature. Subsequently the gel is washed on a sinter funnel with a 10-fold volume of the phosphate buffer containing 150 mmolar NaCl and with 5 volumes of the phosphate buffer containing 2 molar NaCl. Then the gel is equilibrated with an access of 0.1 molar phosphate buffer pH 7.0.

The gel is then transferred into a chromatographic column having a dimension of diameter to gel bed height of 1:4. By determining the peptide concentration the solution of the incubation supernatant after separation from the gel and the washing solutions the amount of peptide coupled to the affinity matrix is calculated. The coupling rate is 85%.

The gel is subsequently used to purify vWF from a Factor VIII (FVIII)/vWF complex. A FVIII/vWF complex concentrate is produced according to US 4,814,435 containing vWF in a concentration of 260 U vWF:Ag/ml and a specific activity of 13.5 U vWF:Ag/mg Protein. The concentrate is diluted with 20 mM phosphate buffer pH 7.0 to a final vWF concentration of 6 U vWF:Ag/mL. A volume of 20 ml of this solution is subjected to the affinity column with immobilized peptide described above. After washing the column with 10 ml of the phosphate buffer the vWF specifically bound to the peptide ligand is eluted by a linear gradient from 0-2 mol/l NaCl in phosphate buffer at a flow rate of 1 ml / minute. Fractions of 1 ml are collected and their optical density is determined at 280 nm. All fractions are measured for their content of vWF antigen determined by a specific ELISA method (Asserachrom vWF, Boehringer, Mannheim). Measurement showed a specific peak of vWF eluting from the peptide at a NaCl concentration of 100 mmol/l, while most of the protein measured by UV-absorption eluted prior to the vWF fraction with the washing buffer. The vWF containing fractions are pooled and measured for vWF activity. The vWF in this pool had a specific activity, of 95 U vWF/mg protein and is essentially free from other proteins.

### Example 5:

### Anti-vWF-cp polypeptide antibodies

The peptide with the sequence AAGGILHLELLV (SEQ.ID.No. 1) is synthesized and purified as described in example 4. The peptide is then used to immunize 3 months old BALB/c mice with the following protocol: A primary subcutaneous injection of 100 µg peptide antigen emulsified in Freund's complete adjuvant in 100 µl followed by intra-peritoneal boosts of 100 µg peptide antigen in phosphate buffered saline at monthly intervals.

The anti-peptide titer is tested by routine ELISA method using purified peptide as screening antigen. After the final boost the spleens are taken from the mice for cell fusion. Cell fusion is carried out according to a standard protocol originally described by Köhler et al. (1975, Nature 256:495-497). Anti-peptide antibodies producing hybridoma cell lines are screened by standard techniques with the purified peptide as screening antigen essentially based on a conventional ELISA methodology. After cloning a cell line can be isolated with a high expression level of an antibody specific for the screening peptide with the sequence AAGGILHLELLV. This cell line is cultured on serum-free culture medium and grown to high density. The supernatant of the cell culture is harvested by centrifugation to remove cells and the monoclonal antibody containing supernatant is concentrated by ultra-diafiltration and conditioned for further use.

The monoclonal antibody obtained had a high selectivity for the vWF cleaving protease as described by Furlan et al. (1996, Blood 87: 4223-4234). This monoclonal antibody is immobilized to a polystyrene ELISA plate in a carbonate/bi-carbonate buffer, 0.05 molar, pH 9.6, at a concentration of 5 µg immunoglobuline/ml overnight (16 hours) at 4°C, with each 100 µl of coating solution per well. The coating solution is removed from the wells and replaced by a solution of bovine serum albumin (BSA) at a concentration of 100 µg/ml at a volume of 100 µL per well, for 2 hours. The BSA solution is removed and the wells are washed with phosphate buffered saline. The pre-coated plates are then incubated with either samples of platelet poor plasma from healthy human plasma donors or platelet poor plasma from patients with an unclear diagnosis of either thrombotic thrombocytopenic purpura (TTP) or hemolytic uremic syndrome (HUS). After incubation of the plasma samples with the antibody coated ELISA plates as in a routine sandwich ELISA system, after 3 hours the plasma is removed from the wells. Wells are washed with phosphate buffered saline and incubated with the monoclonal antibody directed against the peptide with the sequence AAGGILHLELLV, conjugated with horse radish peroxidase following the method of Wilson et al. (1978. In: Immunofluorescence and Related Staining Techniques; Knapp, W. Holubar, K. and Wick, G (eds.), Elsevier/North Holland, Amsterdam, 215) and detected by the OPT reagent as described by Cathy et al, (1989. ELISA and related enzyme immunoassays, In: Antibodies II a practical approach. Cathy D (ed.), IRL Press Eynsham Oxford England, 97).

Based on the level of the samples from the healthy human plasma donors a normal range is established. Plasmas from patients with HUS had a vWF protease activity equivalent to healthy humans while patients with TTP had a decreased protease activity as confirmed by an assay based on a different assay principle as described in WO 00/50904.

### Example 6:

### Cloning of the vWF cleaving protease gene

Human salivary gland poly A+ RNA is purchased from Clontech. First strand cDNA is obtained using Expand reverse transcriptase (Hoffmann La Roche) and oligo d(T) primer according to the manufacturer's instructions. PCR is performed using 5' CGGCGGGATCCTACACCTGG 3' (SEQ.ID.NO. 10) and 5'AATGGTGACTCCCAGGTCGA 3' (SEQ.ID.NO. 11) as primers with 10 ng of salivary gland cDNA as template and 10 U of Hot Star Taq polymerase (Qiagen). The thermal cycling parameters are an initial incubation at 94° C for 15 minutes followed by 45 cycles of 94° C (50 sec), 50° C (50 sec), 72° C (2 min). PCR products are directly sequenced in both directions using the BigDye Terminator Cycle Sequencing Ready Reaction Kit (PerkinElmer Life Science).

The obtained DNA sequence is used to scan the genomic data base using BLAST (basic local alignment search tool) programs and matched to the chromosome 9 clone RP11-224N20. DNA sequence is translated to amino acids sequence using ExPASy proteomic tools. The DNA and translated amino acid sequence corresponding to 4 putative exons of chromosome 9q34 is shown in Fig. 5.

RT-PCR fragments encompassing either the pre-prosequence or the mature domains of the vWF cleaving protease are cloned into vector pDrive (Qiagen) by PCR cloning. For amplification of mature vWF-cp the primer #6601 (5'-AGCGGTCTCTATGGCTGCAGGCGGCATCCTACACC-3') (SEQ.ID. NO. 30) and #6617 (5'-AGCCTCGAGCTGGCCAGACACGGAACAAAT-3') (SEQ.ID.NO. 31) are used. The resulting DNA-fragment is ligated to the T-overlaps of vector pDrive. The construct comprising mature vWF-cp is called pFP H251/8. For amplification of pre-prosequence of vWF-cp the primer #66128 (5'-GGCGAATTCATGCACCAGCGTCACCCCCG- 3') (SEQ. ID. NO. 32) and #6787 (5'-ACAGCATTAAACTAAGCCGCC-3') (SEQ.ID.NO. 33) are used. The resulting DNA-fragment is inserted into vector pDrive. The resulting construct is called pFP H262-35/14.

### Example 7

### Expression of vWF cleaving protease

The eukaryotic expression vector pcDNA3.1(+) (Invitrogen) is used for the expression of the full-length vWF cleaving protease (vWF-cp) under control of the human CMV promoter.

A 4.12 kB EcoRI/XhoI fragment is excised from the plasmid pFP H251/8, the fragment encompassing the cDNA sequence encoding the mature vWF-cp from nucleotide nt 223 to 4284 (without pre-pro-sequence from nt 1 to nt 222) and inserted into the EcoRI/XhoI sites of pcDNA3.1(+). To complete the 5' -end of the cDNA to add the leader and propeptide sequence, a PCR is performed using plasmid pFP H262-35/14 which contains the cDNA sequence of vWF-cp from nt -10 (10 nt uptstream from start codon ATG) to nt +824 in vector pDrive (Qiagen) as template and direct primer 6839 (5'-GATCGAATTCGCCGGCCACCATGCACCAGCGTCACCC CCG- 3') (SEQ.ID.NO. 34), harbouring the Kozak consensus sequence (underlined) for the optimal context for the recognition of the start codon, and reverse primer 3113 (5'- CGGATAACAATTTCACACAGG-3') (SEQ.ID.NO. 35), which binds in the lacZ region of pDrive vector. PCR is accomplished under standard reaction conditions using HotStar DNA polymerase (Qiagen) and Q-solution (Qiagen). The amplified fragment comprising nt -10 to +824 is cut with EcoRI and AscI and used to replace the EcoRI/AscI fragment of the incomplete clone containing only the mature sequence of vWF-cp. The latter construct encoding the complete sequence of vWF-cp called pCMV-vWFcp is further used for transfection and expression studies in mammalian cells.

For expression of vWF-cp, SKHep cells (ATCC) cells are transiently transfected with plasmid pCMV-vWFcp and, in parallel as a control with parental vector cDNA3.1(+), using Lipofectamine 2000 (Life Technologies, Inc). Transfected cells are cultivated under standard conditions in DMEM/HAM's F12(1:1) medium (GIBCO) with 10% fetal calf serum, at a temperature of 37°C and 5% CO₂.

vWF-cp expression is analysed by Western blot, whereby samples of conditioned medium and cell harvest are subjected to SDS-PAGE on a 4%stacking/6% separation gel and Westernblot analysis is performed using anti-vWF-cp TTP patient plasma containing polyclonal anti-vWF-cp antibodies and goat-anti-hu-IgG (Fab-specific AB, SIGMA). VWF-cp specifics bands are visualized by BCIP/NBT detection.

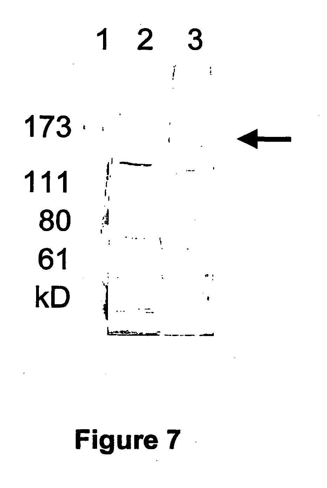

VWF-cp could be detected in sample of the cell lysate and seems to be mainly cell-associated. The results of the Westernblot analysis is shown in Figure 7, showing specific protein band of vWF-cp at about molecular weight of about 170kD reacting with patient sera.

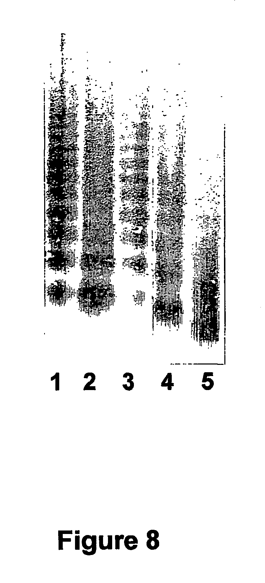

The cell lysate of the vWF-cp expressing cells are further tested for vWF-cp activity according to the method as described by Furlan et al. (1996. Blood 87: 4235-4244) and vWF multimer analysis is performed in 1% SDS-agarose gel as described by Ruggeri et al. (1981. Blood 57: 1140-1143) (Fig. 8). The biological activity of the vWF-cp expressed in mammalian cells could be clearly shown as high molecular weight vWF is degradated to vWF molecules having lower molecular weight (Fig. 8, lane 5).

## Claims

1. A polypeptide exhibiting vWF-cp activity comprising the amino acid sequence AAGGILHLELLV, wherein the vWF-cp activity is defined by (1) cleaving vWF at the peptide bond 842Tyr-843Met, (2) having direct proteolytic activity which converts vWF having a singlet structure to vWF having a satellite structure, and (3) retaining activity in the presence, individually, of the serine protease inhibitor diisopropyl fluorophosphate (DFP) and the calpain protease inhibitor carbobenzyloxy (Z) peptidyl diazomethylketone (Z-Leu-Leu-Tyr-CHN₂).

2. The polypeptide of Claim 1 having a molecular weight as determined by SDS PAGE under reducing conditions of about 180 kD, about 170 kD, about 160 kD or about 120 kD.

3. The polypeptide of Claim 1 or 2 comprising the amino acid sequence of SEQ ID No. 2 or SEQ ID No. 3.

4. The polypeptide of any of Claims 1 to 3, wherein said polypeptide retains vWF cleaving protease activity in the presence of serine protease inhibitor and a calpain protease inhibitor.

5. The polypeptide of any of Claims 1 to 4, wherein the polypeptide is expressed by a host cell transformed with a vector carrying a vWF-cp polypeptide-encoding nucleic acid.

6. The polypeptide of any preceding claim which is substantially pure.

7. The polypeptide of any preceding claim which is isolated.

8. A composition comprising a polypeptide according to any of the claims 1 - 7.

9. A composition according to claim 8 further comprising a divalent metal ion selected from the group of Ca²⁺, Sr²⁺ and Ba²⁺.

10. A composition according to claim 8 or 9 comprising clusterin or an analog or derivative thereof having clusterin activity.

11. A nucleic acid molecule comprising a nucleic acid sequence encoding a polypeptide according to any of the claims 1 to 7.

12. An expression vector comprising a nucleic acid molecule according to claim 11.

13. A host cell comprising an expression vector according to claim 12.

14. A method for production of a vWF-cp protease polypeptide comprising the steps of
- growing a host cell according to claim 13 in a nutrient medium under conditions to express said polypeptide
- harvesting said polypeptide expressed and
- isolating said polypeptide.

15. A method for production of anti-vWF cp polypeptide antibodies wherein an animal is immunized with a vWF-cp polypeptide according to any of the claims 1 to 7 and anti-vWF-cp polypeptide antibodies are isolated from said animal.

16. A method for purification of vWF -cp polypeptide comprising the step of contacting a solution comprising vwF -cp polypeptide with vWF protease polypeptide binding molecule selected from the group of antibodies, single chain antibodies and Fab- or Fab'2-fragments under conditions whereby vHF -cp polypeptide is bound to said vWF -cp polypeptide binding molecule, selectively eluting vWF -cp polypeptide from said binding molecule and recovering purified vWF-cp polypeptide.

17. A method for detection of vWF -cp polypeptide in a sample, wherein a solution suspected to contain vWF - cp is contacted with a vWF-cp polypeptide binding molecule selected from the group of antibodies, single chain antibodies and Fab- or Fab'2-fragments to allow the formation of an vWF -cp polypeptide/ binding molecule complex and detection of said complex.

18. Use of a vWF-cp polypeptide according to any of the claims 1 to 7 for the production of a preparation for prophylaxis and therapy of thrombosis and thromboembolic disease.

19. Use according to claim 18, wherein the thromboembolic disease is thrombotic thrombocytic purpura (TTP), Henoch-Schönlein purpura, preeclampsia, neonatal thrombocytopenia or hemolyticuremic syndrome.

## Patentansprüche

1. Polypeptid, welches vWF-cp-Aktivität zeigt, umfassend die Aminosäuresequenz AAGGILHLELLV, wobei die vWF-cp Aktivität durch
(1) das Abspalten von vWF an der Peptid-Bindung 842Tyr-843Met,
(2) das Aufweisen direkter protolytischer Aktivität, welche vWF mit einer Singulet-Struktur zu vWF mit einer Satellitenstruktur umwandelt, und
(3) das Beibehalten von Aktivität in der Gegenwart, jeweils für sich, des Serin-Protease-Inhibitors Diisopropylfluorphosphat (DFP) und des Calpain-Protease-Inhibitors Carbobenzyloxy (Z) Peptidyldiazomethylketon (Z-Leu-Leu-Tyr-CHN₂), definiert ist.

2. Polypeptid nach Anspruch 1, mit einem Molekulargewicht von etwa 180 kD, etwa 170 kD, etwa 160 kD oder etwa 120 kD, wie durch SDS PAGE unter reduzierenden Bedingungen ermittelt.

3. Polypeptid nach Anspruch 1 oder 2, umfassend die Aminosäuresequenz von SEQ ID Nr. 2 oder SEQ ID Nr. 3.

4. Polypeptid nach einem der Ansprüche 1 bis 3, wobei das Polypeptid vWF-Spaltungsproteaseaktivität in der Gegenwart von Serin-Protease-Inhibitor und einem Calpain-Protease-Inhibitor beibehält.

5. Polypeptid nach einem der Ansprüche 1 bis 4, wobei das Polypeptid durch eine Wirtszelle exprimiert ist, welche mit einem Vektor transformiert ist, der eine vWF-cp-Polypeptid kodierende Nukleinsäure trägt.

6. Polypeptid nach jedem vorhergehenden Anspruch, welches im wesentlichen rein ist.

7. Polypeptid nach jedem vorhergehenden Anspruch, welches isoliert ist.

8. Zusammensetzung, umfassend ein Polypeptid nach einem der Ansprüche 1 bis 7.

9. Zusammensetzung nach Anspruch 8, weiter umfassend ein zweiwertiges Metallion, ausgewählt aus der Gruppe von Ca²⁺, Sr²⁺ und Ba²⁺.

10. Zusammensetzung nach Anspruch 8 oder 9, umfassend Clusterin oder ein Analogon oder Derivat davon, welches Clusterinaktivität aufweist.

11. Nukleinsäuremolekül, umfassend eine Nukleinsäuresequenz, welche ein Polypeptid nach einem der Ansprüche 1 bis 7 kodiert.

12. Expressionsvektor, umfassend ein Nukleinsäuremolekül nach Anspruch 11.

13. Wirtszelle, umfassend ein Expressionsvektor nach Anspruch 12.

14. Verfahren zur Herstellung eines vWF-cp-Proteasepolypeptids, umfassend die Schritte:
des Züchtens einer Wirtszelle nach Anspruch 13 in einem Nährmedium unter Bedingungen, um das Polypeptid zu exprimieren,
des Erntens des exprimierten Polypeptids und
des Isolierens des Polypeptids.

15. Verfahren zur Herstellung von Anti-vWF-cp-Polypeptidantikörpern, wobei ein Tier mit einem vWF-cp-Polypeptid nach einem der Ansprüche 1 bis 7 immunisiert wird, und Anti-vWF-cp-Polypeptidantikörper von dem Tier isoliert werden.

16. Verfahren zur Reinigung eines vWF-cp-Polypeptids, umfassend den Schritt des Inkontaktbringens einer Lösung, welche vWF-cp-Polypeptid umfaßt, mit einem vWF-Proteasepolypeptid bindenden Molekül, ausgewählt aus der Gruppe von Antikörpern, einkettigen Antikörpern, und Fab- oder Fab'2-Fragmenten, unter Bedingungen, wobei vWF-cp-Polypeptid an das vWF-cp-Polypeptid bindende Molekül gebunden wird, des selektiven Eluierens von vWF-cp-Polypeptid aus dem bindenden Molekül und das Gewinnen von gereinigten vWF-cp-Polypeptid.

17. Verfahren zum Nachweis von vWF-cp-Polypeptid in einer Probe, wobei eine Lösung, von der vermutet wird, daß sie vWF-cp enthält, mit einem vWF-cp-Polypeptid bindenden Molekül, ausgewählt aus der Gruppe von Antikörpern, einkettigen Antikörpern und Fab- oder Fab'2-Fragmenten, in Kontakt gebracht wird, um die Bildung eines vWF-cp-Polypeptid/bindendes Molekül-Komplex und den Nachweis des Komplexes zu erlauben.

18. Verwendung eines vWF-cp-Polypeptids nach einem der Ansprüche 1 bis 7, zur Herstellung einer Zubereitung für die Prophylaxe oder Therapie von Thrombose und thromboembolischer Erkrankung.

19. Verwendung nach Anspruch 18, wobei die thromboembolische Erkrankung thrombotische thrombozytische Purpura (TTP), Henoch-Schönlein-Purpura, Präeklampsie, neonatale Thrombozytopenie oder hämolytisch-urämisches Syndrom ist.

## Revendications

1. Polypeptide présentant une activité de vWF-cp, comprenant la séquence d'acides aminés AAGGILHLELLV, dans lequel l'activité de vWF-cp est définie par (1) un clivage de vWF au niveau de la liaison peptidique 842Tyr-843Met, (2) ayant une activité protéolytique directe qui transforme vWF ayant une structure de singulet en vWF ayant une structure de satellite, et (3) maintenant l'activité en présence, individuellement, de l'inhibiteur de sérine-protéase fluorophosphate de diisopropyle (DFP) et de l'inhibiteur de calpaïne-protéase carbobenzyloxy (Z)-peptidyl-diazométhylcétone (Z-Leu-Leu-Tyr-CHN₂).

2. Polypeptide selon la revendication 1, ayant un poids moléculaire, tel que déterminé par SGS PAGE dans des conditions réductrices, d'environ 180 kD, d'environ 170 kD, d'environ 160 kD ou d'environ 120 kD.

3. Polypeptide selon la revendication 1 ou 2, comprenant la séquence d'acides aminés de SEQ ID No. 2 ou SEQ ID No. 3.

4. Polypeptide selon l'une quelconque des revendications 1 à 3, ledit polypeptide conservant une activité de protéase clivant vWF en présence d'un inhibiteur de sérine-protéase et d'un inhibiteur de calpaïne-protéase.

5. Polypeptide selon l'une quelconque des revendications 1 à 4, ledit polypeptide étant exprimé par une cellule hôte transformée avec un vecteur portant un acide nucléique codant pour un polypeptide vWF-cp.

6. Polypeptide selon l'une quelconque des revendications précédentes, qui est sensiblement pur.

7. Polypeptide selon l'une quelconque des revendications précédentes, qui est isolé.

8. Composition comprenant un polypeptide selon l'une quelconque des revendications 1 à 7.

9. Composition selon la revendication 8, comprenant en outre un ion de métal divalent choisi dans l'ensemble constitué de Ca²⁺, Sr²⁺ et Ba²⁺.

10. Composition selon la revendication 8 ou 9, comprenant de la clustérine ou un analogue ou un dérivé de celle-ci ayant une activité de clustérine.

11. Molécule d'acide nucléique comprenant une séquence d'acide nucléique codant pour un polypeptide selon l'une quelconque des revendications 1 à 7.

12. Vecteur d'expression comprenant une molécule d'acide nucléique selon la revendication 11.

13. Cellule hôte comprenant un vecteur d'expression selon la revendication 12.

14. Procédé de production d'un polypeptide vWF-cp-protéase, comprenant les étapes consistant à :
- faire croître une cellule hôte selon la revendication 13 dans un milieu nutritif dans des conditions qui expriment ledit polypeptide
- récolter ledit polypeptide exprimé et
- isoler ledit polypeptide.

15. Procédé de production d'anticorps anti-polypeptide vWF-cp, dans lequel un animal est immunisé avec un polypeptide vWF-cp selon l'une quelconque des revendications 1 à 7 et on isole les anticorps anti-polypeptide vWF-cp dudit animal.

16. Procédé de purification d'un polypeptide vWF-cp, comprenant les étapes consistant à amener une solution comprenant un polypeptide vWF-cp au contact d'une molécule de liaison de polypeptide de vWF-protéase, choisie dans l'ensemble constitué d'anticorps, d'anticorps à chaîne simple et de fragments Fab ou Fab'2, dans des conditions au moyen desquelles le polypeptide vWF-cp est lié à ladite molécule de liaison du polypeptide vWF-cp, à éluer sélectivement le polypeptide vWF-cp à partir de ladite molécule de liaison et à recueillir le polypeptide vWF-cp purifié.

17. Procédé de détection d'un polypeptide vWF-cp dans un échantillon, dans lequel on amène une solution susceptible de contenir vWF-cp au contact d'une molécule de liaison de polypeptide vWF-cp, choisie dans l'ensemble constitué d'anticorps, d'anticorps à chaîne simple et de fragments Fab ou Fab'2, pour permettre la formation d'un complexe de polypeptide vWF-cp / molécule de liaison et la détection dudit complexe.

18. Utilisation d'un polypeptide vWF-cp selon l'une quelconque des revendications 1 à 7 pour la production d'une préparation pour la prophylaxie et la thérapie d'une thrombose et d'une maladie thrombo-embolique.

19. Utilisation selon la revendication 18, dans laquelle la maladie thrombo-embolique est le purpura thrombocytaire thrombotique (TTP), le purpura de Schönlein-Henoch, l'éclampsisme, la thrombocytopénie néonatale ou le syndrome hémolytique et urémique.


### SEQUENCE LISTING

<110> Baxter AG
<120> von Willebrand factor(vWf)cleaving protease polypeptide, nucleic acid encoding the polypeptide and use of polypeptide
<130> FA247
<140>
   <141>
<150> 09/721,254
   <151> 2000-11-22
<150> 09/833,328
   <151> 2001-04-12
<160> 35
<170> PatentIn Ver. 2.1
<210> 1
   <211> 12
   <212> PRT
   <213> Homo sapiens
<400> 1
   Ala Ala Gly Gly Ile Leu His Leu Glu Leu Leu Val
    1               5                  10
<210> 2
   <211> 15
   <212> PRT
   <213> Homo sapiens
<400> 2
   Ala Ala Gly Gly Ile Leu His Leu Glu Leu Leu Val Ala Val Gly
    1               5                  10                  15
<210> 3
   <211> 148
   <212> PRT
   <213> Homo sapiens
<400> 3
   Ala Ala Gly Gly Ile Leu His Leu Glu Leu Leu Val Ala Val Gly Pro
    1               5                  10                  15
   Asp Val Phe Gln Ala His Gln Glu Asp Thr Glu Arg Tyr Val Leu Thr
               20                  25                  30
   Asn Leu Asn Ile Gly Ala Glu Leu Leu Arg Asp Pro Ser Leu Gly Ala
           35                  40                  45
   Gln Phe Arg Val His Leu Val Lys Met Val Ile Leu Thr Glu Pro Glu
   50 55 60
   Gly Ala Pro Asn Ile Thr Ala Asn Leu Thr Ser Ser Leu Leu Ser Val
   65 70 75 80
   Cys Gly Trp Ser Gln Thr Ile Asn Pro Glu Asp Asp Thr Asp Pro Gly
   85 90 95
   His Ala Asp Leu Val Leu Tyr Ile Thr Arg Phe Asp Leu Glu Leu Pro
   100 105 110
   Asp Gly Asn Arg Gln Val Arg Gly Val Thr Gln Leu Gly Gly Ala Cys
   115 120 125
   Ser Pro Thr Trp Ser Cys Leu Ile Thr Glu Asp Thr Gly Phe Asp Leu
   130 135 140
   Gly Val Thr Ile
   145
<210> 4
   <211> 1353
   <212> PRT
   <213> Homo sapiens
<400> 4
   Ala Ala Gly Gly Ile Leu His Leu Glu Leu Leu Val Ala Val Gly Pro
   1 5 10 15
   Asp Val Phe Gln Ala His Gln Glu Asp Thr Glu Arg Tyr Val Leu Thr
   20 25 30
   Asn Leu Asn Ile Gly Ala Glu Leu Leu Arg Asp Pro Ser Leu Gly Ala
   35 40 45
   Gln Phe Arg Val His Leu Val Lys Met Val Ile Leu Thr Glu Pro Glu
   50 55 60
   Gly Ala Pro Asn Ile Thr Ala Asn Leu Thr Ser Ser Leu Leu Ser Val
   65 70 75 80
   Cys Gly Trp Ser Gln Thr Ile Asn Pro Glu Asp Asp Thr Asp Pro Gly
   85 90 95
   His Ala Asp Leu Val Leu Tyr Ile Thr Arg Phe Asp Leu Glu Leu Pro
   100 105 110
   Asp Gly Asn Arg Gln Val Arg Gly Val Thr Gln Leu Gly Gly Ala Cys
   115 120 125
   Ser Pro Thr Trp Ser Cys Leu Ile Thr Glu Asp Thr Gly Phe Asp Leu
   130 135 140
   Gly Val Thr Ile Ala His Glu Ile Gly His Ser Phe Gly Leu Glu His
   145                 150                 155                 160
   Asp Gly Ala Pro Gly Ser Gly Cys Gly Pro Ser Gly His Val Met Ala
                   165                 170                 175
   Ser Asp Gly Ala Ala Pro Arg Ala Gly Leu Ala Trp Ser Pro Cys Ser
               180                 185                 190
   Arg Arg Gln Leu Leu Ser Leu Leu Ser Ala Gly Arg Ala Arg Cys Val
           195                 200                 205
   Trp Asp Pro Pro Arg Pro Gln Pro Gly Ser Ala Gly His Pro Pro Asp
       210                 215                 220
   Ala Gln Pro Gly Leu Tyr Tyr Ser Ala Asn Glu Gln Cys Arg Val Ala
   225                 230                 235                 240
   Phe Gly Pro Lys Ala Val Ala Cys Thr Phe Ala Arg Glu His Leu Asp
                   245                 250                 255
   Met Cys Gln Ala Leu Ser Cys His Thr Asp Pro Leu Asp Gln Ser Ser
               260                 265                 270
   Cys Ser Arg Leu Leu Val Pro Leu Leu Asp Gly Thr Glu Cys Gly Val
           275                 280                 285
   Glu Lys Trp Cys Ser Lys Gly Arg Cys Arg Ser Leu Val Glu Leu Thr
       290                 295                 300
   Pro Ile Ala Ala Val His Gly Arg Trp Ser Ser Trp Gly Pro Arg Ser
   305                 310                 315                 320
   Pro Cys Ser Arg Ser Cys Gly Gly Gly Val Val Thr Arg Arg Arg Gln
                   325                 330                 335
   Cys Asn Asn Pro Arg Pro Ala Phe Gly Gly Arg Ala Cys Val Gly Ala
               340                 345                 350
   Asp Leu Gln Ala Glu Met Cys Asn Thr Gln Ala Cys Glu Lys Thr Gln
           355                 360                 365
   Leu Glu Phe Met Ser Gln Gln Cys Ala Arg Thr Asp Gly Gln Pro Leu
       370                 375                 380
   Arg Ser Ser Pro Gly Gly Ala Ser Phe Tyr His Trp Gly Ala Ala Val
   385                 390                 395                 400
   Pro His Ser Gln Gly Asp Ala Leu Cys Arg His Met Cys Arg Ala Ile
                   405                 410                 415
   Gly Glu Ser Phe Ile Met Lys Arg Gly Asp Ser Phe Leu Asp Gly Thr
               420                 425                 430
   Arg Cys Met Pro Ser Gly Pro Arg Glu Asp Gly Thr Leu Ser Leu Cys
           435                 440                 445
   Val Ser Gly Ser Cys Arg Thr Phe Gly Cys Asp Gly Arg Met Asp Ser
       450                 455                 460
   Gln Gln Val Trp Asp Arg Cys Gln Val Cys Gly Gly Asp Asn Ser Thr
   465                 470                 475                 480
   Cys Ser Pro Arg Lys Gly Ser Phe Thr Ala Gly Arg Ala Arg Glu Tyr
                   485                 490                 495
   Val Thr Phe Leu Thr Val Thr Pro Asn Leu Thr Ser Val Tyr Ile Ala
               500                 505                 510
   Asn His Arg Pro Leu Phe Thr His Leu Ala Val Arg Ile Gly Gly Arg
           515                 520                 525
   Tyr Val Val Ala Gly Lys Met Ser Ile Ser Pro Asn Thr Thr Tyr Pro
       530                 535                 540
   Ser Leu Leu Glu Asp Gly Arg Val Glu Tyr Arg Val Ala Leu Thr Glu
   545                 550                 555                 560
   Asp Arg Leu Pro Arg Leu Glu Glu Ile Arg Ile Trp Gly Pro Leu Gln
                   565                 570                 575
   Glu Asp Ala Asp Ile Gln Val Tyr Arg Arg Tyr Gly Glu Glu Tyr Gly
               580                 585                 590
   Asn Leu Thr Arg Pro Asp Ile Thr Phe Thr Tyr Phe Gln Pro Lys Pro
           595                 600                 605
   Arg Gln Ala Trp Val Trp Ala Ala Val Arg Gly Pro Cys Ser Val Ser
       610                 615                 620
   Cys Gly Ala Gly Leu Arg Trp Val Asn Tyr Ser Cys Leu Asp Gln Ala
   625                 630                 635                 640
   Arg Lys Glu Leu Val Glu Thr Val Gln Cys Gln Gly Ser Gln Gln Pro
                   645                 650                 655
   Pro Ala Trp Pro Glu Ala Cys Val Leu Glu Pro Cys Pro Pro Tyr Trp
               660                 665                 670
   Ala Val Gly Asp Phe Gly Pro Cys Ser Ala Ser Cys Gly Gly Gly Leu
           675                 680                 685
   Arg Glu Arg Pro Val Arg Cys Val Glu Ala Gln Gly Ser Leu Leu Lys
       690                 695                 700
   Thr Leu Pro Pro Ala Arg Cys Arg Ala Gly Ala Gln Gln Pro Ala Val
   705                 710                 715                 720
   Ala Leu Glu Thr Cys Asn Pro Gln Pro Cys Pro Ala Arg Trp Glu Val
                   725                 730                 735
   Ser Glu Pro Ser Ser Cys Thr Ser Ala Gly Gly Ala Gly Leu Ala Leu
               740                 745                 750
   Glu Asn Glu Thr Cys Val Pro Gly Ala Asp Gly Leu Glu Ala Pro Val
   755 760 765
   Thr Glu Gly Pro Gly Ser Val Asp Glu Lys Leu Pro Ala Pro Glu Pro
   770 775 780
   Cys Val Gly Met Ser Cys Pro Pro Gly Trp Gly His Leu Asp Ala Thr
   785 790 795 800
   Ser Ala Gly Glu Lys Ala Pro Ser Pro Trp Gly Ser Ile Arg Thr Gly
   805 810 815
   Ala Gln Ala Ala His Val Trp Thr Pro Ala Ala Gly Ser Cys Ser Val
   820 825 830
   Ser Cys Gly Arg Gly Leu Met Glu Leu Arg Phe Leu Cys Met Asp Ser
   835 840 845
   Ala Leu Arg Val Pro Val Gln Glu Glu Leu Cys Gly Leu Ala Ser Lys
   850 855 860
   Pro Gly Ser Arg Arg Glu Val Cys Gln Ala Val Pro Cys Pro Ala Arg
   865 870 875 880
   Trp Gln Tyr Lys Leu Ala Ala Cys Ser Val Ser Cys Gly Arg Gly Val
   885 890 895
   Val Arg Arg Ile Leu Tyr Cys Ala Arg Ala His Gly Glu Asp Asp Gly
   900 905 910
   Glu Glu Ile Leu Leu Asp Thr Gln Cys Gln Gly Leu Pro Arg Pro Glu
   915 920 925
   Pro Gln Glu Ala Cys Ser Leu Glu Pro Cys Pro Pro Arg Trp Lys Val
   930 935 940
   Met Ser Leu Gly Pro Cys Ser Ala Ser Cys Gly Leu Gly Thr Ala Arg
   945 950 955 960
   Arg Ser Val Ala Cys Val Gln Leu Asp Gln Gly Gln Asp Val Glu Val
   965 970 975
   Asp Glu Ala Ala Cys Ala Ala Leu Val Arg Pro Glu Ala Ser Val Pro
   980 985 990
   Cys Leu Ile Ala Asp Cys Thr Tyr Arg Trp His Val Gly Thr Trp Met
   995 1000 1005
   Glu Cys Ser Val Ser Cys Gly Asp Gly Ile Gln Arg Arg Arg Asp Thr
   1010 1015 1020
   Cys Leu Gly Pro Gln Ala Gln Ala Pro Val Pro Ala Asp Phe Cys Gln
   1025 1030 1035 1040
   His Leu Pro Lys Pro Val Thr Val Arg Gly Cys Trp Ala Gly Pro Cys
   1045 1050 1055
   Val Gly Gln Gly Thr Pro Ser Leu Val Pro His Glu Glu Ala Ala Ala
   1060 1065 1070
   Pro Gly Arg Thr Thr Ala Thr Pro Ala Gly Ala Ser Leu Glu Trp Ser
   1075 1080 1085
   Gln Ala Arg Gly Leu Leu Phe Ser Pro Ala Pro Gln Pro Arg Arg Leu
   1090 1095 1100
   Leu Pro Gly Pro Gln Glu Asn Ser Val Gln Ser Ser Ala Cys Gly Arg
   1105 1110 1115 1120
   Gln His Leu Glu Pro Thr Gly Thr Ile Asp Met Arg Gly Pro Gly Gln
   1125 1130 1135
   Ala Asp Cys Ala Val Ala Ile Gly Arg Pro Leu Gly Glu Val Val Thr
   1140 1145 1150
   Leu Arg Val Leu Glu Ser Ser Leu Asn Cys Ser Ala Gly Asp Met Leu
   1155 1160 1165
   Leu Leu Trp Gly Arg Leu Thr Trp Arg Lys Met Cys Arg Lys Leu Leu
   1170 1175 1180
   Asp Met Thr Phe Ser Ser Lys Thr Asn Thr Leu Val Val Arg Gln Arg
   1185 1190 1195 1200
   Cys Gly Arg Pro Gly Gly Gly Val Leu Leu Arg Tyr Gly Ser Gln Leu
   1205 1210 1215
   Ala Pro Glu Thr Phe Tyr Arg Glu Cys Asp Met Gln Leu Phe Gly Pro
   1220 1225 1230
   Trp Gly Glu Ile Val Ser Pro Ser Leu Ser Pro Ala Thr Ser Asn Ala
   1235 1240 1245
   Gly Gly Cys Arg Leu Phe Ile Asn Val Ala Pro His Ala Arg Ile Ala
   1250 1255 1260
   Ile His Ala Leu Ala Thr Asn Met Gly Ala Gly Thr Glu Gly Ala Asn
   1265 1270 1275 1280
   Ala Ser Tyr Ile Leu Ile Arg Asp Thr His Ser Leu Arg Thr Thr Ala
   1285 1290 1295
   Phe His Gly Gln Gln Val Leu Tyr Trp Glu Ser Glu Ser Ser Gln Ala
   1300 1305 1310
   Glu Met Glu Phe Ser Glu Gly Phe Leu Lys Ala Gln Ala Ser Leu Arg
   1315 1320 1325
   Gly Gln Tyr Trp Thr Leu Gln Ser Trp Val Pro Glu Met Gln Asp Pro
   1330 1335 1340
   Gln Ser Trp Lys Gly Lys Glu Gly Thr
   1345 1350
<210> 5
   <211> 1427
   <212> PRT
   <213> Homo sapiens
<400> 5
   Met His Gln Arg His Pro Arg Ala Arg Cys Pro Pro Leu Cys Val Ala
   1               5                   10                  15
   Gly Ile Leu Ala Cys Gly Phe Leu Leu Gly Cys Trp Gly Pro Ser His
               20                  25                  30
   Phe Gln Gln Ser Cys Leu Gln Ala Leu Glu Pro Gln Ala Val Ser Ser
           35                  40                  45
   Tyr Leu Ser Pro Gly Ala Pro Leu Lys Gly Arg Pro Pro Ser Pro Gly
       50                  55                  60
   Phe Gln Arg Gln Arg Gln Arg Gln Arg Arg Ala Ala Gly Gly Ile Leu
   65                  70                  75                  80
   His Leu Glu Leu Leu Val Ala Val Gly Pro Asp Val Phe Gln Ala His
                   85                  90                  95
   Gln Glu Asp Thr Glu Arg Tyr Val Leu Thr Asn Leu Asn Ile Gly Ala
               100                 105                 110
   Glu Leu Leu Arg Asp Pro Ser Leu Gly Ala Gln Phe Arg Val His Leu
           115                 120                 125
   Val Lys Met Val Ile Leu Thr Glu Pro Glu Gly Ala Pro Asn Ile Thr
       130                 135                 140
   Ala Asn Leu Thr Ser Ser Leu Leu Ser Val Cys Gly Trp Ser Gln Thr
   145                 150                 155                 160
   Ile Asn Pro Glu Asp Asp Thr Asp Pro Gly His Ala Asp Leu Val Leu
                   165                 170                 175
   Tyr Ile Thr Arg Phe Asp Leu Glu Leu Pro Asp Gly Asn Arg Gln Val
               180                 185                 190
   Arg Gly Val Thr Gln Leu Gly Gly Ala Cys Ser Pro Thr Trp Ser Cys
           195                 200                 205
   Leu Ile Thr Glu Asp Thr Gly Phe Asp Leu Gly Val Thr Ile Ala His
       210                 215                 220
   Glu Ile Gly His Ser Phe Gly Leu Glu His Asp Gly Ala Pro Gly Ser
   225                 230                 235                 240
   Gly Cys Gly Pro Ser Gly His Val Met Ala Ser Asp Gly Ala Ala Pro
   245 250 255
   Arg Ala Gly Leu Ala Trp Ser Pro Cys Ser Arg Arg Gln Leu Leu Ser
   260 265 270
   Leu Leu Ser Ala Gly Arg Ala Arg Cys Val Trp Asp Pro Pro Arg Pro
   275 280 285
   Gln Pro Gly Ser Ala Gly His Pro Pro Asp Ala Gln Pro Gly Leu Tyr
   290 295 300
   Tyr Ser Ala Asn Glu Gln Cys Arg Val Ala Phe Gly Pro Lys Ala Val
   305 310 315 320
   Ala Cys Thr Phe Ala Arg Glu His Leu Asp Met Cys Gln Ala Leu Ser
   325 330 335
   Cys His Thr Asp Pro Leu Asp Gln Ser Ser Cys Ser Arg Leu Leu Val
   340 345 350
   Pro Leu Leu Asp Gly Thr Glu Cys Gly Val Glu Lys Trp Cys Ser Lys
   355 360 365
   Gly Arg Cys Arg Ser Leu Val Glu Leu Thr Pro Ile Ala Ala Val His
   370 375 380
   Gly Arg Trp Ser Ser Trp Gly Pro Arg Ser Pro Cys Ser Arg Ser Cys
   385 390 395 400
   Gly Gly Gly Val Val Thr Arg Arg Arg Gln Cys Asn Asn Pro Arg Pro
   405 410 415
   Ala Phe Gly Gly Arg Ala Cys Val Gly Ala Asp Leu Gln Ala Glu Met
   420 425 430
   Cys Asn Thr Gln Ala Cys Glu Lys Thr Gln Leu Glu Phe Met Ser Gln
   435 440 445
   Gln Cys Ala Arg Thr Asp Gly Gln Pro Leu Arg Ser Ser Pro Gly Gly
   450 455 460
   Ala Ser Phe Tyr His Trp Gly Ala Ala Val Pro His Ser Gln Gly Asp
   465 470 475 480
   Ala Leu Cys Arg His Met Cys Arg Ala Ile Gly Glu Ser Phe Ile Met
   485 490 495
   Lys Arg Gly Asp Ser Phe Leu Asp Gly Thr Arg Cys Met Pro Ser Gly
   500 505 510
   Pro Arg Glu Asp Gly Thr Leu Ser Leu Cys Val Ser Gly Ser Cys Arg
   515 520 525
   Thr Phe Gly Cys Asp Gly Arg Met Asp Ser Gln Gln Val Trp Asp Arg
   530 535 540
   Cys Gln Val Cys Gly Gly Asp Asn Ser Thr Cys Ser Pro Arg Lys Gly
   545 550 555 560
   Ser Phe Thr Ala Gly Arg Ala Arg Glu Tyr Val Thr Phe Leu Thr Val
   565 570 575
   Thr Pro Asn Leu Thr Ser Val Tyr Ile Ala Asn His Arg Pro Leu Phe
   580 585 590
   Thr His Leu Ala Val Arg Ile Gly Gly Arg Tyr Val Val Ala Gly Lys
   595 600 605
   Met Ser Ile Ser Pro Asn Thr Thr Tyr Pro Ser Leu Leu Glu Asp Gly
   610 615 620
   Arg Val Glu Tyr Arg Val Ala Leu Thr Glu Asp Arg Leu Pro Arg Leu
   625 630 635 640
   Glu Glu Ile Arg Ile Trp Gly Pro Leu Gln Glu Asp Ala Asp Ile Gln
   645 650 655
   Val Tyr Arg Arg Tyr Gly Glu Glu Tyr Gly Asn Leu Thr Arg Pro Asp
   660 665 670
   Ile Thr Phe Thr Tyr Phe Gln Pro Lys Pro Arg Gln Ala Trp Val Trp
   675 680 685
   Ala Ala Val Arg Gly Pro Cys Ser Val Ser Cys Gly Ala Gly Leu Arg
   690 695 700
   Trp Val Asn Tyr Ser Cys Leu Asp Gln Ala Arg Lys Glu Leu Val Glu
   705 710 715 720
   Thr Val Gln Cys Gln Gly Ser Gln Gln Pro Pro Ala Trp Pro Glu Ala
   725 730 735
   Cys Val Leu Glu Pro Cys Pro Pro Tyr Trp Ala Val Gly Asp Phe Gly
   740 745 750
   Pro Cys Ser Ala Ser Cys Gly Gly Gly Leu Arg Glu Arg Pro Val Arg
   755 760 765
   Cys Val Glu Ala Gln Gly Ser Leu Leu Lys Thr Leu Pro Pro Ala Arg
   770 775 780
   Cys Arg Ala Gly Ala Gln Gln Pro Ala Val Ala Leu Glu Thr Cys Asn
   785 790 795 800
   Pro Gln Pro Cys Pro Ala Arg Trp Glu Val Ser Glu Pro Ser Ser Cys
   805 810 815
   Thr Ser Ala Gly Gly Ala Gly Leu Ala Leu Glu Asn Glu Thr Cys Val
   820 825 830
   Pro Gly Ala Asp Gly Leu Glu Ala Pro Val Thr Glu Gly Pro Gly Ser
   835 840 845
   Val Asp Glu Lys Leu Pro Ala Pro Glu Pro Cys Val Gly Met Ser Cys
       850                 855                 860
   Pro Pro Gly Trp Gly His Leu Asp Ala Thr Ser Ala Gly Glu Lys Ala
   865                 870                 875                 880
   Pro Ser Pro Trp Gly Ser Ile Arg Thr Gly Ala Gln Ala Ala His Val
                   885                 890                 895
   Trp Thr Pro Ala Ala Gly Ser Cys Ser Val Ser Cys Gly Arg Gly Leu
               900                 905                 910
   Met Glu Leu Arg Phe Leu Cys Met Asp Ser Ala Leu Arg Val Pro Val
           915                 920                 925
   Gln Glu Glu Leu Cys Gly Leu Ala Ser Lys Pro Gly Ser Arg Arg Glu
       930                 935                 940
   Val Cys Gln Ala Val Pro Cys Pro Ala Arg Trp Gln Tyr Lys Leu Ala
   945                 950                 955                 960
   Ala Cys Ser Val Ser Cys Gly Arg Gly Val Val Arg Arg Ile Leu Tyr
                   965                 970                 975
   Cys Ala Arg Ala His Gly Glu Asp Asp Gly Glu Glu Ile Leu Leu Asp
               980                 985                 990
   Thr Gln Cys Gln Gly Leu Pro Arg Pro Glu Pro Gln Glu Ala Cys Ser
           995                 1000                1005
   Leu Glu Pro Cys Pro Pro Arg Trp Lys Val Met Ser Leu Gly Pro Cys
      1010                1015                1020
   Ser Ala Ser Cys Gly Leu Gly Thr Ala Arg Arg Ser Val Ala Cys Val
   1025                1030                1035                1040
   Gln Leu Asp Gln Gly Gln Asp Val Glu Val Asp Glu Ala Ala Cys Ala
                   1045                1050                1055
   Ala Leu Val Arg Pro Glu Ala Ser Val Pro Cys Leu Ile Ala Asp Cys
               1060                1065                1070
   Thr Tyr Arg Trp His Val Gly Thr Trp Met Glu Cys Ser Val Ser Cys
           1075                1080                1085
   Gly Asp Gly Ile Gln Arg Arg Arg Asp Thr Cys Leu Gly Pro Gln Ala
      1090                1095                1100
   Gln Ala Pro Val Pro Ala Asp Phe Cys Gln His Leu Pro Lys Pro Val
   1105                1110                1115                1120
   Thr Val Arg Gly Cys Trp Ala Gly Pro Cys Val Gly Gln Gly Thr Pro
                   1125                1130                1135
   Ser Leu Val Pro His Glu Glu Ala Ala Ala Pro Gly Arg Thr Thr Ala
               1140                1145                1150
   Thr Pro Ala Gly Ala Ser Leu Glu Trp Ser Gln Ala Arg Gly Leu Leu
   1155 1160 1165
   Phe Ser Pro Ala Pro Gln Pro Arg Arg Leu Leu Pro Gly Pro Gln Glu
   1170 1175 1180
   Asn Ser Val Gln Ser Ser Ala Cys Gly Arg Gln His Leu Glu Pro Thr
   1185 1190 1195 1200
   Gly Thr Ile Asp Met Arg Gly Pro Gly Gln Ala Asp Cys Ala Val Ala
   1205 1210 1215
   Ile Gly Arg Pro Leu Gly Glu Val Val Thr Leu Arg Val Leu Glu Ser
   1220 1225 1230
   Ser Leu Asn Cys Ser Ala Gly Asp Met Leu Leu Leu Trp Gly Arg Leu
   1235 1240 1245
   Thr Trp Arg Lys Met Cys Arg Lys Leu Leu Asp Met Thr Phe Ser Ser
   1250 1255 1260
   Lys Thr Asn Thr Leu Val Val Arg Gln Arg Cys Gly Arg Pro Gly Gly
   1265 1270 1275 1280
   Gly Val Leu Leu Arg Tyr Gly Ser Gln Leu Ala Pro Glu Thr Phe Tyr
   1285 1290 1295
   Arg Glu Cys Asp Met Gln Leu Phe Gly Pro Trp Gly Glu Ile Val Ser
   1300 1305 1310
   Pro Ser Leu Ser Pro Ala Thr Ser Asn Ala Gly Gly Cys Arg Leu Phe
   1315 1320 1325
   Ile Asn Val Ala Pro His Ala Arg Ile Ala Ile His Ala Leu Ala Thr
   1330 1335 1340
   Asn Met Gly Ala Gly Thr Glu Gly Ala Asn Ala Ser Tyr Ile Leu Ile
   1345 1350 1355 1360
   Arg Asp Thr His Ser Leu Arg Thr Thr Ala Phe His Gly Gln Gln Val
   1365 1370 1375
   Leu Tyr Trp Glu Ser Glu Ser Ser Gln Ala Glu Met Glu Phe Ser Glu
   1380 1385 1390
   Gly Phe Leu Lys Ala Gln Ala Ser Leu Arg Gly Gln Tyr Trp Thr Leu
   1395 1400 1405
   Gln Ser Trp Val Pro Glu Met Gln Asp Pro Gln Ser Trp Lys Gly Lys
   1410 1415 1420
   Glu Gly Thr
   1425
<210> 6
   <211> 4585
   <212> DNA
   <213> Homo sapiens
<400> 6
   cccattccat actgaccaga ttcccagtca ccaaggcccc ctctcactcc gctccactcc 60
   tcgggctggc tctcctgagg atgcaccagc gtcacccccg ggcaagatgc cctcccctct 120
   gtgtggccgg aatccttgcc tgtggctttc tcctgggctg ctggggaccc tcccatttcc 180
   agcagagttg tcttcaggct ttggagccac aggccgtgtc ttcttacttg agccctggtg 240
   ctcccttaaa aggccgccct ccttcccctg gcttccagag gcagaggcag aggcagaggc 300
   gggctgcagg cggcatccta cacctggagc tgctggtggc cgtgggcccc gatgtcttcc 360
   aggctcacca ggaggacaca gagcgctatg tgctcaccaa cctcaacatc ggggcagaac 420
   tgcttcggga cccgtccctg ggggctcagt ttcgggtgca cctggtgaag atggtcattc 480
   tgacagagcc tgagggtgct ccaaatatca cagccaacct cacctcgtcc ctgctgagcg 540
   tctgtgggtg gagccagacc atcaaccctg aggacgacac ggatcctggc catgctgacc 600
   tggtcctcta tatcactagg tttgacctgg agttgcctga tggtaaccgg caggtgcggg 660
   gcgtcaccca gctgggcggt gcctgctccc caacctggag ctgcctcatt accgaggaca 720
   ctggcttcga cctgggagtc accattgccc atgagattgg gcacagcttc ggcctggagc 780
   acgacggcgc gcccggcagc ggctgcggcc ccagcggaca cgtgatggct tcggacggcg 840
   ccgcgccccg cgccggcctc gcctggtccc cctgcagccg ccggcagctg ctgagcctgc 900
   tcagcgcagg acgggcgcgc tgcgtgtggg acccgccgcg gcctcaaccc gggtccgcgg 960
   ggcacccgcc ggatgcgcag cctggcctct actacagcgc caacgagcag tgccgcgtgg 1020
   ccttcggccc caaggctgtc gcctgcacct tcgccaggga gcacctggat atgtgccagg 1080
   ccctctcctg ccacacagac ccgctggacc aaagcagctg cagccgcctc ctcgttcctc 1140
   tcctggatgg gacagaatgt ggcgtggaga agtggtgctc caagggtcgc tgccgctccc 1200
   tggtggagct gacccccata gcagcagtgc atgggcgctg gtctagctgg ggtccccgaa 1260
   gtccttgctc ccgctcctgc ggaggaggtg tggtcaccag gaggcggcag tgcaacaacc 1320
   ccagacctgc ctttgggggg cgtgcatgtg ttggtgctga cctccaggcc gagatgtgca 1380
   acactcaggc ctgcgagaag acccagctgg agttcatgtc gcaacagtgc gccaggaccg 1440
   acggccagcc gctgcgctcc tcccctggcg gcgcctcctt ctaccactgg ggtgctgctg 1500
   taccacacag ccaaggggat gctctgtgca gacacatgtg ccgggccatt ggcgagagct 1560
   tcatcatgaa gcgtggagac agcttcctcg atgggacccg gtgtatgcca agtggccccc 1620
   gggaggacgg gaccctgagc ctgtgtgtgt cgggcagctg caggacattt ggctgtgatg 1680
   gtaggatgga ctcccagcag gtatgggaca ggtgccaggt gtgtggtggg gacaacagca 1740
   cgtgcagccc acggaagggc tctttcacag ctggcagagc gagagaatat gtcacgtttc 1800
   tgacagttac ccccaacctg accagtgtct acattgccaa ccacaggcct ctcttcacac 1860
   acttggcggt gaggatcgga gggcgctatg tcgtggctgg gaagatgagc atctccccta 1920
   acaccaccta cccctccctc ctggaggatg gtcgtgtcga gtacagagtg gccctcaccg 1980
   aggaccggct gccccgcctg gaggagatcc gcatctgggg acccctccag gaagatgctg 2040
   acatccaggt ttacaggcgg tatggcgagg agtatggcaa cctcacccgc ccagacatca 2100
   ccttcaccta cttccagcct aagccacggc aggcctgggt gtgggccgct gtgcgtgggc 2160
   cctgctcggt gagctgtggg gcagggctgc gctgggtaaa ctacagctgc ctggaccagg 2220
   ccaggaagga gttggtggag actgtccagt gccaagggag ccagcagcca ccagcgtggc 2280
   cagaggcctg cgtgctcgaa ccctgccctc cctactgggc ggtgggagac ttcggcccat 2340
   gcagcgcctc ctgtgggggt ggcctgcggg agcggccagt gcgctgcgtg gaggcccagg 2400
   gcagcctcct gaagacattg cccccagccc ggtgcagagc aggggcccag cagccagctg 2460
   tggcgctgga aacctgcaac ccccagccct gccctgccag gtgggaggtg tcagagccca 2520
   gctcatgcac atcagctggt ggagcaggcc tggccttgga gaacgagacc tgtgtgccag 2580
   gggcagatgg cctggaggct ccagtgactg aggggcctgg ctccgtagat gagaagctgc 2640
   ctgcccctga gccctgtgtc gggatgtcat gtcctccagg ctggggccat ctggatgcca 2700
   cctctgcagg ggagaaggct ccctccccat ggggcagcat caggacgggg gctcaagctg 2760
   cacacgtgtg gacccctgcg gcagggtcgt gctccgtctc ctgcgggcga ggtctgatgg 2820
   agctgcgttt cctgtgcatg gactctgccc tcagggtgcc tgtccaggaa gagctgtgtg 2880
   gcctggcaag caagcctggg agccggcggg aggtctgcca ggctgtcccg tgccctgctc 2940
   ggtggcagta caagctggcg gcctgcagcg tgagctgtgg gagaggggtc gtgcggagga 3000
   tcctgtattg tgcccgggcc catggggagg acgatggtga ggagatcctg ttggacaccc 3060
   agtgccaggg gctgcctcgc ccggaacccc aggaggcctg cagcctggag ccctgcccac 3120
   ctaggtggaa agtcatgtcc cttggcccat gttcggccag ctgtggcctt ggcactgcta 3180
   gacgctcggt ggcctgtgtg cagctcgacc aaggccagga cgtggaggtg gacgaggcgg 3240
   cctgtgcggc gctggtgcgg cccgaggcca gtgtcccctg tctcattgcc gactgcacct 3300
   accgctggca tgttggcacc tggatggagt gctctgtttc ctgtggggat ggcatccagc 3360
   gccggcgtga cacctgcctc ggaccccagg cccaggcgcc tgtgccagct gatttctgcc 3420
   agcacttgcc caagccggtg actgtgcgtg gctgctgggc tgggccctgt gtgggacagg 3480
   gtacgcccag cctggtgccc cacgaagaag ccgctgctcc aggacggacc acagccaccc 3540
   ctgctggtgc ctccctggag tggtcccagg cccggggcct gctcttctcc ccggctcccc 3600
   agcctcggcg gctcctgccc gggccccagg aaaactcagt gcagtccagt gcctgtggca 3660
   ggcagcacct tgagccaaca ggaaccattg acatgcgagg cccagggcag gcagactgtg 3720
   cagtggccat tgggcggccc ctcggggagg tggtgaccct ccgcgtcctt gagagttctc 3780
   tcaactgcag tgcgggggac atgttgctgc tttggggccg gctcacctgg aggaagatgt 3840
   gcaggaagct gttggacatg actttcagct ccaagaccaa cacgctggtg gtgaggcagc 3900
   gctgcgggcg gccaggaggt ggggtgctgc tgcggtatgg gagccagctt gctcctgaaa 3960
   ccttctacag agaatgtgac atgcagctct ttgggccctg gggtgaaatc gtgagcccct 4020
   cgctgagtcc agccacgagt aatgcagggg gctgccggct cttcattaat gtggctccgc 4080
   acgcacggat tgccatccat gccctggcca ccaacatggg cgctgggacc gagggagcca 4140
   atgccagcta catcttgatc cgggacaccc acagcttgag gaccacagcg ttccatgggc 4200
   agcaggtgct ctactgggag tcagagagca gccaggctga gatggagttc agcgagggct 4260
   tcctgaaggc tcaggccagc ctgcggggcc agtactggac cctccaatca tgggtaccgg 4320
   agatgcagga ccctcagtcc tggaagggaa aggaaggaac ctgagggtca ttgaacattt 4380
   gttccgtgtc tggccagccc tggagggttg acccctggtc tcagtgcttt ccaattcgaa 4440
   ctttttccaa tcttaggtat ctactttaga gtcttctcca atgtccaaaa ggctaggggg 4500
   ttggaggtgg ggactctgga aaagcagccc ccatttcctc gggtaccaat aaataaaaca 4560
   tgcaggcaaa aaaaaaaaaa aaaaa                                       4585
<210> 7
   <211> 4284
   <212> DNA
   <213> Homo sapiens
<220>
   <221> CDS
   <222> (1)..(4284)
<400> 7
   atg cac cag cgt cac ccc cgg gca aga tgc cct ccc ctc tgt gtg gcc      48
   Met His Gln Arg His Pro Arg Ala Arg Cys Pro Pro Leu Cys Val Ala
   1               5                   10                  15
   gga atc ctt gcc tgt ggc ttt ctc ctg ggc tgc tgg gga ccc tcc cat      96
   Gly Ile Leu Ala Cys Gly Phe Leu Leu Gly Cys Trp Gly Pro Ser His
               20                  25                  30
   ttc cag cag agt tgt ctt cag gct ttg gag cca cag gcc gtg tct tct      144
   Phe Gln Gln Ser Cys Leu Gln Ala Leu Glu Pro Gln Ala Val Ser Ser
           35                  40                  45
   tac ttg agc cct ggt gct ccc tta aaa ggc cgc cct cct tcc cct ggc      192
   Tyr Leu Ser Pro Gly Ala Pro Leu Lys Gly Arg Pro Pro Ser Pro Gly
       50                  55                  60
   ttc cag agg cag agg cag agg cag agg cgg gct gca ggc ggc atc cta      240
   Phe Gln Arg Gln Arg Gln Arg Gln Arg Arg Ala Ala Gly Gly Ile Leu
   65                  70                  75                  80
   cac ctg gag ctg ctg gtg gcc gtg ggc ccc gat gtc ttc cag gct cac      288
   His Leu Glu Leu Leu Val Ala Val Gly Pro Asp Val Phe Gln Ala His
                   85                  90                  95
   cag gag gac aca gag cgc tat gtg ctc acc aac ctc aac atc ggg gca      336
   Gln Glu Asp Thr Glu Arg Tyr Val Leu Thr Asn Leu Asn Ile Gly Ala
               100                 105                 110
   gaa ctg ctt cgg gac ccg tcc ctg ggg gct cag ttt cgg gtg cac ctg      384
   Glu Leu Leu Arg Asp Pro Ser Leu Gly Ala Gln Phe Arg Val His Leu
           115                 120                 125
   gtg aag atg gtc att ctg aca gag cct gag ggt gct cca aat atc aca      432
   Val Lys Met Val Ile Leu Thr Glu Pro Glu Gly Ala Pro Asn Ile Thr
       130                 135                 140
   gcc aac ctc acc tcg tcc ctg ctg agc gtc tgt ggg tgg agc cag acc     480
   Ala Asn Leu Thr Ser Ser Leu Leu Ser Val Cys Gly Trp Ser Gln Thr
   145                 150                 155                 160
   atc aac cct gag gac gac acg gat cct ggc cat gct gac ctg gtc ctc     528
   Ile Asn Pro Glu Asp Asp Thr Asp Pro Gly His Ala Asp Leu Val Leu
                   165                 170                 175
   tat atc act agg ttt gac ctg gag ttg cct gat ggt aac cgg cag gtg     576
   Tyr Ile Thr Arg Phe Asp Leu Glu Leu Pro Asp Gly Asn Arg Gln Val
               180                 185                 190
   cgg ggc gtc acc cag ctg ggc ggt gcc tgc tcc cca acc tgg agc tgc     624
   Arg Gly Val Thr Gln Leu Gly Gly Ala Cys Ser Pro Thr Trp Ser Cys
           195                 200                 205
   ctc att acc gag gac act ggc ttc gac ctg gga gtc acc att gcc cat     672
   Leu Ile Thr Glu Asp Thr Gly Phe Asp Leu Gly Val Thr Ile Ala His
       210                 215                 220
   gag att ggg cac agc ttc ggc ctg gag cac gac ggc gcg ccc ggc agc     720
   Glu Ile Gly His Ser Phe Gly Leu Glu His Asp Gly Ala Pro Gly Ser
   225                 230                 235                 240
   ggc tgc ggc ccc agc gga cac gtg atg gct tcg gac ggc gcc gcg ccc     768
   Gly Cys Gly Pro Ser Gly His Val Met Ala Ser Asp Gly Ala Ala Pro
                   245                 250                 255
   cgc gcc ggc ctc gcc tgg tcc ccc tgc agc cgc cgg cag ctg ctg agc     816
   Arg Ala Gly Leu Ala Trp Ser Pro Cys Ser Arg Arg Gln Leu Leu Ser
               260                 265                 270
   ctg ctc agc gca gga cgg gcg cgc tgc gtg tgg gac ccg ccg cgg cct     864
   Leu Leu Ser Ala Gly Arg Ala Arg Cys Val Trp Asp Pro Pro Arg Pro
           275                 280                 285
   caa ccc ggg tcc gcg ggg cac ccg ccg gat gcg cag cct ggc ctc tac     912
   Gln Pro Gly Ser Ala Gly His Pro Pro Asp Ala Gln Pro Gly Leu Tyr
       290                 295                 300
   tac agc gcc aac gag cag tgc cgc gtg gcc ttc ggc ccc aag gct gtc     960
   Tyr Ser Ala Asn Glu Gln Cys Arg Val Ala Phe Gly Pro Lys Ala Val
   305                 310                 315                 320
   gcc tgc acc ttc gcc agg gag cac ctg gat atg tgc cag gcc ctc tcc     1008
   Ala Cys Thr Phe Ala Arg Glu His Leu Asp Met Cys Gln Ala Leu Ser
                   325                 330                 335
   tgc cac aca gac ccg ctg gac caa agc agc tgc agc cgc ctc ctc gtt     1056
   Cys His Thr Asp Pro Leu Asp Gln Ser Ser Cys Ser Arg Leu Leu Val
               340                 345                 350
   cct ctc ctg gat ggg aca gaa tgt ggc gtg gag aag tgg tgc tcc aag     1104
   Pro Leu Leu Asp Gly Thr Glu Cys Gly Val Glu Lys Trp Cys Ser Lys
           355                 360                 365
   ggt cgc tgc cgc tcc ctg gtg gag ctg acc ccc ata gca gca gtg cat    1152
   Gly Arg Cys Arg Ser Leu Val Glu Leu Thr Pro Ile Ala Ala Val His
       370                 375                 380
   ggg cgc tgg tct agc tgg ggt ccc cga agt cct tgc tcc cgc tcc tgc    1200
   Gly Arg Trp Ser Ser Trp Gly Pro Arg Ser Pro Cys Ser Arg Ser Cys
   385                 390                 395                 400
   gga gga ggt gtg gtc acc agg agg cgg cag tgc aac aac ccc aga cct    1248
   Gly Gly Gly Val Val Thr Arg Arg Arg Gln Cys Asn Asn Pro Arg Pro
                   405                 410                 415
   gcc ttt ggg ggg cgt gca tgt gtt ggt gct gac ctc cag gcc gag atg    1296
   Ala Phe Gly Gly Arg Ala Cys Val Gly Ala Asp Leu Gln Ala Glu Met
               420                 425                 430
   tgc aac act cag gcc tgc gag aag acc cag ctg gag ttc atg tcg caa    1344
   Cys Asn Thr Gln Ala Cys Glu Lys Thr Gln Leu Glu Phe Met Ser Gln
           435                 440                 445
   cag tgc gcc agg acc gac ggc cag ccg ctg cgc tcc tcc cct ggc ggc    1392
   Gln Cys Ala Arg Thr Asp Gly Gln Pro Leu Arg Ser Ser Pro Gly Gly
       450                 455                 460
   gcc tcc ttc tac cac tgg ggt gct gct gta cca cac agc caa ggg gat    1440
   Ala Ser Phe Tyr His Trp Gly Ala Ala Val Pro His Ser Gln Gly Asp
   465                 470                 475                 480
   gct ctg tgc aga cac atg tgc cgg gcc att ggc gag agc ttc atc atg    1488
   Ala Leu Cys Arg His Met Cys Arg Ala Ile Gly Glu Ser Phe Ile Met
                   485                 490                 495
   aag cgt gga gac agc ttc ctc gat ggg acc cgg tgt atg cca agt ggc    1536
   Lys Arg Gly Asp Ser Phe Leu Asp Gly Thr Arg Cys Met Pro Ser Gly
               500                 505                 510
   ccc cgg gag gac ggg acc ctg agc ctg tgt gtg tcg ggc agc tgc agg    1584
   Pro Arg Glu Asp Gly Thr Leu Ser Leu Cys Val Ser Gly Ser Cys Arg
           515                 520                 525
   aca ttt ggc tgt gat ggt agg atg gac tcc cag cag gta tgg gac agg    1632
   Thr Phe Gly Cys Asp Gly Arg Met Asp Ser Gln Gln Val Trp Asp Arg
       530                 535                 540
   tgc cag gtg tgt ggt ggg gac aac agc acg tgc agc cca cgg aag ggc    1680
   Cys Gln Val Cys Gly Gly Asp Asn Ser Thr Cys Ser Pro Arg Lys Gly
   545                 550                 555                 560
   tct ttc aca gct ggc aga gcg aga gaa tat gtc acg ttt ctg aca gtt    1728
   Ser Phe Thr Ala Gly Arg Ala Arg Glu Tyr Val Thr Phe Leu Thr Val
                   565                 570                 575
   acc ccc aac ctg acc agt gtc tac att gcc aac cac agg cct ctc ttc    1776
   Thr Pro Asn Leu Thr Ser Val Tyr Ile Ala Asn His Arg Pro Leu Phe
               580                 585                 590
   aca cac ttg gcg gtg agg atc gga ggg cgc tat gtc gtg gct ggg aag     1824
   Thr His Leu Ala Val Arg Ile Gly Gly Arg Tyr Val Val Ala Gly Lys
           595                 600                 605
   atg agc atc tcc cct aac acc acc tac ccc tcc ctc ctg gag gat ggt     1872
   Met Ser Ile Ser Pro Asn Thr Thr Tyr Pro Ser Leu Leu Glu Asp Gly
       610                 615                 620
   cgt gtc gag tac aga gtg gcc ctc acc gag gac cgg ctg ccc cgc ctg     1920
   Arg Val Glu Tyr Arg Val Ala Leu Thr Glu Asp Arg Leu Pro Arg Leu
   625                 630                 635                 640
   gag gag atc cgc atc tgg gga ccc ctc cag gaa gat gct gac atc cag     1968
   Glu Glu Ile Arg Ile Trp Gly Pro Leu Gln Glu Asp Ala Asp Ile Gln
                   645                 650                 655
   gtt tac agg cgg tat ggc gag gag tat ggc aac ctc acc cgc cca gac     2016
   Val Tyr Arg Arg Tyr Gly Glu Glu Tyr Gly Asn Leu Thr Arg Pro Asp
               660                 665                 670
   atc acc ttc acc tac ttc cag cct aag cca cgg cag gcc tgg gtg tgg     2064
   Ile Thr Phe Thr Tyr Phe Gln Pro Lys Pro Arg Gln Ala Trp Val Trp
           675                 680                 685
   gcc gct gtg cgt ggg ccc tgc tcg gtg agc tgt ggg gca ggg ctg cgc     2112
   Ala Ala Val Arg Gly Pro Cys Ser Val Ser Cys Gly Ala Gly Leu Arg
       690                 695                 700
   tgg gta aac tac agc tgc ctg gac cag gcc agg aag gag ttg gtg gag     2160
   Trp Val Asn Tyr Ser Cys Leu Asp Gln Ala Arg Lys Glu Leu Val Glu
   705                 710                 715                 720
   act gtc cag tgc caa ggg agc cag cag cca cca gcg tgg cca gag gcc     2208
   Thr Val Gln Cys Gln Gly Ser Gln Gln Pro Pro Ala Trp Pro Glu Ala
                   725                 730                 735
   tgc gtg ctc gaa ccc tgc cct ccc tac tgg gcg gtg gga gac ttc ggc     2256
   Cys Val Leu Glu Pro Cys Pro Pro Tyr Trp Ala Val Gly Asp Phe Gly
               740                 745                 750
   cca tgc agc gcc tcc tgt ggg ggt ggc ctg cgg gag cgg cca gtg cgc     2304
   Pro Cys Ser Ala Ser Cys Gly Gly Gly Leu Arg Glu Arg Pro Val Arg
           755                 760                 765
   tgc gtg gag gcc cag ggc agc ctc ctg aag aca ttg ccc cca gcc cgg     2352
   Cys Val Glu Ala Gln Gly Ser Leu Leu Lys Thr Leu Pro Pro Ala Arg
       770                 775                 780
   tgc aga gca ggg gcc cag cag cca gct gtg gcg ctg gaa acc tgc aac     2400
   Cys Arg Ala Gly Ala Gln Gln Pro Ala Val Ala Leu Glu Thr Cys Asn
   785                 790                 795                 800
   ccc cag ccc tgc cct gcc agg tgg gag gtg tca gag ccc agc tca tgc     2448
   Pro Gln Pro Cys Pro Ala Arg Trp Glu Val Ser Glu Pro Ser Ser Cys
                   805                 810                 815
   aca tca gct ggt gga gca ggc ctg gcc ttg gag aac gag acc tgt gtg    2496
   Thr Ser Ala Gly Gly Ala Gly Leu Ala Leu Glu Asn Glu Thr Cys Val
               820                 825                 830
   cca ggg gca gat ggc ctg gag gct cca gtg act gag ggg cct ggc tcc    2544
   Pro Gly Ala Asp Gly Leu Glu Ala Pro Val Thr Glu Gly Pro Gly Ser
           835                 840                 845
   gta gat gag aag ctg cct gcc cct gag ccc tgt gtc ggg atg tca tgt    2592
   Val Asp Glu Lys Leu Pro Ala Pro Glu Pro Cys Val Gly Met Ser Cys
       850                 855                 860
   cct cca ggc tgg ggc cat ctg gat gcc acc tct gca ggg gag aag gct    2640
   Pro Pro Gly Trp Gly His Leu Asp Ala Thr Ser Ala Gly Glu Lys Ala
   865                 870                 875                 880
   ccc tcc cca tgg ggc agc atc agg acg ggg gct caa gct gca cac gtg    2688
   Pro Ser Pro Trp Gly Ser Ile Arg Thr Gly Ala Gln Ala Ala His Val
                   885                 890                 895
   tgg acc cct gcg gca ggg tcg tgc tcc gtc tcc tgc ggg cga ggt ctg    2736
   Trp Thr Pro Ala Ala Gly Ser Cys Ser Val Ser Cys Gly Arg Gly Leu
               900                 905                 910
   atg gag ctg cgt ttc ctg tgc atg gac tct gcc ctc agg gtg cct gtc    2784
   Met Glu Leu Arg Phe Leu Cys Met Asp Ser Ala Leu Arg Val Pro Val
           915                 920                 925
   cag gaa gag ctg tgt ggc ctg gca agc aag cct ggg agc cgg cgg gag    2832
   Gln Glu Glu Leu Cys Gly Leu Ala Ser Lys Pro Gly Ser Arg Arg Glu
       930                 935                 940
   gtc tgc cag gct gtc ccg tgc cct gct cgg tgg cag tac aag ctg gcg    2880
   Val Cys Gln Ala Val Pro Cys Pro Ala Arg Trp Gln Tyr Lys Leu Ala
   945                 950                 955                 960
   gcc tgc agc gtg agc tgt ggg aga ggg gtc gtg cgg agg atc ctg tat    2928
   Ala Cys Ser Val Ser Cys Gly Arg Gly Val Val Arg Arg Ile Leu Tyr
                   965                 970                 975
   tgt gcc cgg gcc cat ggg gag gac gat ggt gag gag atc ctg ttg gac    2976
   Cys Ala Arg Ala His Gly Glu Asp Asp Gly Glu Glu Ile Leu Leu Asp
               980                 985                 990
   acc cag tgc cag ggg ctg cct cgc ccg gaa ccc cag gag gcc tgc agc    3024
   Thr Gln Cys Gln Gly Leu Pro Arg Pro Glu Pro Gln Glu Ala Cys Ser
           995                 1000                1005
   ctg gag ccc tgc cca cct agg tgg aaa gtc atg tcc ctt ggc cca tgt    3072
   Leu Glu Pro Cys Pro Pro Arg Trp Lys Val Met Ser Leu Gly Pro Cys
       1010                1015                1020
   tcg gcc agc tgt ggc ctt ggc act gct aga cgc tcg gtg gcc tgt gtg    3120
   Ser Ala Ser Cys Gly Leu Gly Thr Ala Arg Arg Ser Val Ala Cys Val
   1025                1030                1035                1040
   cag ctc gac caa ggc cag gac gtg gag gtg gac gag gcg gcc tgt gcg    3168
   Gln Leu Asp Gln Gly Gln Asp Val Glu Val Asp Glu Ala Ala Cys Ala
                   1045                1050                1055
   gcg ctg gtg cgg ccc gag gcc agt gtc ccc tgt ctc att gcc gac tgc    3216
   Ala Leu Val Arg Pro Glu Ala Ser Val Pro Cys Leu Ile Ala Asp Cys
               1060                1065                1070
   acc tac cgc tgg cat gtt ggc acc tgg atg gag tgc tct gtt tcc tgt    3264
   Thr Tyr Arg Trp His Val Gly Thr Trp Met Glu Cys Ser Val Ser Cys
           1075                1080                1085
   ggg gat ggc atc cag cgc cgg cgt gac acc tgc ctc gga ccc cag gcc    3312
   Gly Asp Gly Ile Gln Arg Arg Arg Asp Thr Cys Leu Gly Pro Gln Ala
       1090                1095                1100
   cag gcg cct gtg cca gct gat ttc tgc cag cac ttg ccc aag ccg gtg    3360
   Gln Ala Pro Val Pro Ala Asp Phe Cys Gln His Leu Pro Lys Pro Val
   1105                1110                1115                1120
   act gtg cgt ggc tgc tgg gct ggg ccc tgt gtg gga cag ggt acg ccc    3408
   Thr Val Arg Gly Cys Trp Ala Gly Pro Cys Val Gly Gln Gly Thr Pro
                   1125                1130                1135
   agc ctg gtg ccc cac gaa gaa gcc gct gct cca gga cgg acc aca gcc    3456
   Ser Leu Val Pro His Glu Glu Ala Ala Ala Pro Gly Arg Thr Thr Ala
               1140                1145                1150
   acc cct gct ggt gcc tcc ctg gag tgg tcc cag gcc cgg ggc ctg ctc    3504
   Thr Pro Ala Gly Ala Ser Leu Glu Trp Ser Gln Ala Arg Gly Leu Leu
           1155                1160                1165
   ttc tcc ccg gct ccc cag cct cgg cgg ctc ctg ccc ggg ccc cag gaa    3552
   Phe Ser Pro Ala Pro Gln Pro Arg Arg Leu Leu Pro Gly Pro Gln Glu
       1170                1175                1180
   aac tca gtg cag tcc agt gcc tgt ggc agg cag cac ctt gag cca aca    3600
   Asn Ser Val Gln Ser Ser Ala Cys Gly Arg Gln His Leu Glu Pro Thr
   1185                1190                1195                1200
   gga acc att gac atg cga ggc cca ggg cag gca gac tgt gca gtg gcc    3648
   Gly Thr Ile Asp Met Arg Gly Pro Gly Gln Ala Asp Cys Ala Val Ala
                   1205                1210                1215
   att ggg cgg ccc ctc ggg gag gtg gtg acc ctc cgc gtc ctt gag agt    3696
   Ile Gly Arg Pro Leu Gly Glu Val Val Thr Leu Arg Val Leu Glu Ser
               1220                1225                1230
   tct ctc aac tgc agt gcg ggg gac atg ttg ctg ctt tgg ggc cgg ctc    3744
   Ser Leu Asn Cys Ser Ala Gly Asp Met Leu Leu Leu Trp Gly Arg Leu
           1235                1240                1245
   acc tgg agg aag atg tgc agg aag ctg ttg gac atg act ttc agc tcc    3792
   Thr Trp Arg Lys Met Cys Arg Lys Leu Leu Asp Met Thr Phe Ser Ser
       1250                1255                1260
   aag acc aac acg ctg gtg gtg agg cag cgc tgc ggg cgg cca gga ggt    3840
   Lys Thr Asn Thr Leu Val Val Arg Gln Arg Cys Gly Arg Pro Gly Gly
   1265                1270                1275                1280
   ggg gtg ctg ctg cgg tat ggg agc cag ctt gct cct gaa acc ttc tac    3888
   Gly Val Leu Leu Arg Tyr Gly Ser Gln Leu Ala Pro Glu Thr Phe Tyr
                   1285                1290                1295
   aga gaa tgt gac atg cag ctc ttt ggg ccc tgg ggt gaa atc gtg agc    3936
   Arg Glu Cys Asp Met Gln Leu Phe Gly Pro Trp Gly Glu Ile Val Ser
               1300                1305                1310
   ccc tcg ctg agt cca gcc acg agt aat gca ggg ggc tgc cgg ctc ttc    3984
   Pro Ser Leu Ser Pro Ala Thr Ser Asn Ala Gly Gly Cys Arg Leu Phe
           1315                1320                1325
   att aat gtg gct ccg cac gca cgg att gcc atc cat gcc ctg gcc acc    4032
   Ile Asn Val Ala Pro His Ala Arg Ile Ala Ile His Ala Leu Ala Thr
       1330                1335                1340
   aac atg ggc gct ggg acc gag gga gcc aat gcc agc tac atc ttg atc    4080
   Asn Met Gly Ala Gly Thr Glu Gly Ala Asn Ala Ser Tyr Ile Leu Ile
   1345                1350                1355                1360
   cgg gac acc cac agc ttg agg acc aca gcg ttc cat ggg cag cag gtg    4128
   Arg Asp Thr His Ser Leu Arg Thr Thr Ala Phe His Gly Gln Gln Val
                   1365                1370                1375
   ctc tac tgg gag tca gag agc agc cag gct gag atg gag ttc agc gag    4176
   Leu Tyr Trp Glu Ser Glu Ser Ser Gln Ala Glu Met Glu Phe Ser Glu
               1380                1385                1390
   ggc ttc ctg aag gct cag gcc agc ctg cgg ggc cag tac tgg acc ctc    4224
   Gly Phe Leu Lys Ala Gln Ala Ser Leu Arg Gly Gln Tyr Trp Thr Leu
           1395                1400                1405
   caa tca tgg gta ccg gag atg cag gac cct cag tcc tgg aag gga aag    4272
   Gln Ser Trp Val Pro Glu Met Gln Asp Pro Gln Ser Trp Lys Gly Lys
       1410                1415                1420
   gaa gga acc tga                                                    4284
   Glu Gly Thr
   1425
<210> 8
   <211> 1427
   <212> PRT
   <213> Homo sapiens
<400> 8
   Met His Gln Arg His Pro Arg Ala Arg Cys Pro Pro Leu Cys Val Ala
    1               5                  10                  15
   Gly Ile Leu Ala Cys Gly Phe Leu Leu Gly Cys Trp Gly Pro Ser His
               20                  25                  30
   Phe Gln Gln Ser Cys Leu Gln Ala Leu Glu Pro Gln Ala Val Ser Ser
           35                  40                  45
   Tyr Leu Ser Pro Gly Ala Pro Leu Lys Gly Arg Pro Pro Ser Pro Gly
       50                  55                  60
   Phe Gln Arg Gln Arg Gln Arg Gln Arg Arg Ala Ala Gly Gly Ile Leu
   65                  70                  75                  80
   His Leu Glu Leu Leu Val Ala Val Gly Pro Asp Val Phe Gln Ala His
                   85                  90                  95
   Gln Glu Asp Thr Glu Arg Tyr Val Leu Thr Asn Leu Asn Ile Gly Ala
               100                 105                 110
   Glu Leu Leu Arg Asp Pro Ser Leu Gly Ala Gln Phe Arg Val His Leu
           115                 120                 125
   Val Lys Met Val Ile Leu Thr Glu Pro Glu Gly Ala Pro Asn Ile Thr
       130                 135                 140
   Ala Asn Leu Thr Ser Ser Leu Leu Ser Val Cys Gly Trp Ser Gln Thr
   145                 150                 155                 160
   Ile Asn Pro Glu Asp Asp Thr Asp Pro Gly His Ala Asp Leu Val Leu
                   165                 170                 175
   Tyr Ile Thr Arg Phe Asp Leu Glu Leu Pro Asp Gly Asn Arg Gln Val
               180                 185                 190
   Arg Gly Val Thr Gln Leu Gly Gly Ala Cys Ser Pro Thr Trp Ser Cys
           195                 200                 205
   Leu Ile Thr Glu Asp Thr Gly Phe Asp Leu Gly Val Thr Ile Ala His
       210                 215                 220
   Glu Ile Gly His Ser Phe Gly Leu Glu His Asp Gly Ala Pro Gly Ser
   225                 230                 235                 240
   Gly Cys Gly Pro Ser Gly His Val Met Ala Ser Asp Gly Ala Ala Pro
                   245                 250                 255
   Arg Ala Gly Leu Ala Trp Ser Pro Cys Ser Arg Arg Gln Leu Leu Ser
               260                 265                 270
   Leu Leu Ser Ala Gly Arg Ala Arg Cys Val Trp Asp Pro Pro Arg Pro
           275                 280                 285
   Gln Pro Gly Ser Ala Gly His Pro Pro Asp Ala Gln Pro Gly Leu Tyr
       290                 295                 300
   Tyr Ser Ala Asn Glu Gln Cys Arg Val Ala Phe Gly Pro Lys Ala Val
   305                 310                 315                 320
   Ala Cys Thr Phe Ala Arg Glu His Leu Asp Met Cys Gln Ala Leu Ser
                   325                 330                 335
   Cys His Thr Asp Pro Leu Asp Gln Ser Ser Cys Ser Arg Leu Leu Val
   340 345 350
   Pro Leu Leu Asp Gly Thr Glu Cys Gly Val Glu Lys Trp Cys Ser Lys
   355 360 365
   Gly Arg Cys Arg Ser Leu Val Glu Leu Thr Pro Ile Ala Ala Val His
   370 375 380
   Gly Arg Trp Ser Ser Trp Gly Pro Arg Ser Pro Cys Ser Arg Ser Cys
   385 390 395 400
   Gly Gly Gly Val Val Thr Arg Arg Arg Gln Cys Asn Asn Pro Arg Pro
   405 410 415
   Ala Phe Gly Gly Arg Ala Cys Val Gly Ala Asp Leu Gln Ala Glu Met
   420 425 430
   Cys Asn Thr Gln Ala Cys Glu Lys Thr Gln Leu Glu Phe Met Ser Gln
   435 440 445
   Gln Cys Ala Arg Thr Asp Gly Gln Pro Leu Arg Ser Ser Pro Gly Gly
   450 455 460
   Ala Ser Phe Tyr His Trp Gly Ala Ala Val Pro His Ser Gln Gly Asp
   465 470 475 480
   Ala Leu Cys Arg His Met Cys Arg Ala Ile Gly Glu Ser Phe Ile Met
   485 490 495
   Lys Arg Gly Asp Ser Phe Leu Asp Gly Thr Arg Cys Met Pro Ser Gly
   500 505 510
   Pro Arg Glu Asp Gly Thr Leu Ser Leu Cys Val Ser Gly Ser Cys Arg
   515 520 525
   Thr Phe Gly Cys Asp Gly Arg Met Asp Ser Gln Gln Val Trp Asp Arg
   530 535 540
   Cys Gln Val Cys Gly Gly Asp Asn Ser Thr Cys Ser Pro Arg Lys Gly
   545 550 555 560
   Ser Phe Thr Ala Gly Arg Ala Arg Glu Tyr Val Thr Phe Leu Thr Val
   565 570 575
   Thr Pro Asn Leu Thr Ser Val Tyr Ile Ala Asn His Arg Pro Leu Phe
   580 585 590
   Thr His Leu Ala Val Arg Ile Gly Gly Arg Tyr Val Val Ala Gly Lys
   595 600 605
   Met Ser Ile Ser Pro Asn Thr Thr Tyr Pro Ser Leu Leu Glu Asp Gly
   610 615 620
   Arg Val Glu Tyr Arg Val Ala Leu Thr Glu Asp Arg Leu Pro Arg Leu
   625 630 635 640
   Glu Glu Ile Arg Ile Trp Gly Pro Leu Gln Glu Asp Ala Asp Ile Gln
   645 650 655
   Val Tyr Arg Arg Tyr Gly Glu Glu Tyr Gly Asn Leu Thr Arg Pro Asp
   660 665 670
   Ile Thr Phe Thr Tyr Phe Gln Pro Lys Pro Arg Gln Ala Trp Val Trp
   675 680 685
   Ala Ala Val Arg Gly Pro Cys Ser Val Ser Cys Gly Ala Gly Leu Arg
   690 695 700
   Trp Val Asn Tyr Ser Cys Leu Asp Gln Ala Arg Lys Glu Leu Val Glu
   705 710 715 720
   Thr Val Gln Cys Gln Gly Ser Gln Gln Pro Pro Ala Trp Pro Glu Ala
   725 730 735
   Cys Val Leu Glu Pro Cys Pro Pro Tyr Trp Ala Val Gly Asp Phe Gly
   740 745 750
   Pro Cys Ser Ala Ser Cys Gly Gly Gly Leu Arg Glu Arg Pro Val Arg
   755 760 765
   Cys Val Glu Ala Gln Gly Ser Leu Leu Lys Thr Leu Pro Pro Ala Arg
   770 775 780
   Cys Arg Ala Gly Ala Gln Gln Pro Ala Val Ala Leu Glu Thr Cys Asn
   785 790 795 800
   Pro Gln Pro Cys Pro Ala Arg Trp Glu Val Ser Glu Pro Ser Ser Cys
   805 810 815
   Thr Ser Ala Gly Gly Ala Gly Leu Ala Leu Glu Asn Glu Thr Cys Val
   820 825 830
   Pro Gly Ala Asp Gly Leu Glu Ala Pro Val Thr Glu Gly Pro Gly Ser
   835 840 845
   Val Asp Glu Lys Leu Pro Ala Pro Glu Pro Cys Val Gly Met Ser Cys
   850 855 860
   Pro Pro Gly Trp Gly His Leu Asp Ala Thr Ser Ala Gly Glu Lys Ala
   865 870 875 880
   Pro Ser Pro Trp Gly Ser Ile Arg Thr Gly Ala Gln Ala Ala His Val
   885 890 895
   Trp Thr Pro Ala Ala Gly Ser Cys Ser Val Ser Cys Gly Arg Gly Leu
   900 905 910
   Met Glu Leu Arg Phe Leu Cys Met Asp Ser Ala Leu Arg Val Pro Val
   915 920 925
   Gln Glu Glu Leu Cys Gly Leu Ala Ser Lys Pro Gly Ser Arg Arg Glu
   930 935 940
   Val Cys Gln Ala Val Pro Cys Pro Ala Arg Trp Gln Tyr Lys Leu Ala
   945 950 955 960
   Ala Cys Ser Val Ser Cys Gly Arg Gly Val Val Arg Arg Ile Leu Tyr
   965 970 975
   Cys Ala Arg Ala His Gly Glu Asp Asp Gly Glu Glu Ile Leu Leu Asp
   980 985 990
   Thr Gln Cys Gln Gly Leu Pro Arg Pro Glu Pro Gln Glu Ala Cys Ser
   995 1000 1005
   Leu Glu Pro Cys Pro Pro Arg Trp Lys Val Met Ser Leu Gly Pro Cys
   1010 1015 1020
   Ser Ala Ser Cys Gly Leu Gly Thr Ala Arg Arg Ser Val Ala Cys Val
   1025 1030 1035 1040
   Gln Leu Asp Gln Gly Gln Asp Val Glu Val Asp Glu Ala Ala Cys Ala
   1045 1050 1055
   Ala Leu Val Arg Pro Glu Ala Ser Val Pro Cys Leu Ile Ala Asp Cys
   1060 1065 1070
   Thr Tyr Arg Trp His Val Gly Thr Trp Met Glu Cys Ser Val Ser Cys
   1075 1080 1085
   Gly Asp Gly Ile Gln Arg Arg Arg Asp Thr Cys Leu Gly Pro Gln Ala
   1090 1095 1100
   Gln Ala Pro Val Pro Ala Asp Phe Cys Gln His Leu Pro Lys Pro Val
   1105 1110 1115 1120
   Thr Val Arg Gly Cys Trp Ala Gly Pro Cys Val Gly Gln Gly Thr Pro
   1125 1130 1135
   Ser Leu Val Pro His Glu Glu Ala Ala Ala Pro Gly Arg Thr Thr Ala
   1140 1145 1150
   Thr Pro Ala Gly Ala Ser Leu Glu Trp Ser Gln Ala Arg Gly Leu Leu
   1155 1160 1165
   Phe Ser Pro Ala Pro Gln Pro Arg Arg Leu Leu Pro Gly Pro Gln Glu
   1170 1175 1180
   Asn Ser Val Gln Ser Ser Ala Cys Gly Arg Gln His Leu Glu Pro Thr
   1185 1190 1195 1200
   Gly Thr Ile Asp Met Arg Gly Pro Gly Gln Ala Asp Cys Ala Val Ala
   1205 1210 1215
   Ile Gly Arg Pro Leu Gly Glu Val Val Thr Leu Arg Val Leu Glu Ser
   1220 1225 1230
   Ser Leu Asn Cys Ser Ala Gly Asp Met Leu Leu Leu Trp Gly Arg Leu
   1235 1240 1245
   Thr Trp Arg Lys Met Cys Arg Lys Leu Leu Asp Met Thr Phe Ser Ser
   1250 1255 1260
   Lys Thr Asn Thr Leu Val Val Arg Gln Arg Cys Gly Arg Pro Gly Gly
   1265 1270 1275 1280
   Gly Val Leu Leu Arg Tyr Gly Ser Gln Leu Ala Pro Glu Thr Phe Tyr
   1285 1290 1295
   Arg Glu Cys Asp Met Gln Leu Phe Gly Pro Trp Gly Glu Ile Val Ser
   1300 1305 1310
   Pro Ser Leu Ser Pro Ala Thr Ser Asn Ala Gly Gly Cys Arg Leu Phe
   1315 1320 1325
   Ile Asn Val Ala Pro His Ala Arg Ile Ala Ile His Ala Leu Ala Thr
   1330 1335 1340
   Asn Met Gly Ala Gly Thr Glu Gly Ala Asn Ala Ser Tyr Ile Leu Ile
   1345 1350 1355 1360
   Arg Asp Thr His Ser Leu Arg Thr Thr Ala Phe His Gly Gln Gln Val
   1365 1370 1375
   Leu Tyr Trp Glu Ser Glu Ser Ser Gln Ala Glu Met Glu Phe Ser Glu
   1380 1385 1390
   Gly Phe Leu Lys Ala Gln Ala Ser Leu Arg Gly Gln Tyr Trp Thr Leu
   1395 1400 1405
   Gln Ser Trp Val Pro Glu Met Gln Asp Pro Gln Ser Trp Lys Gly Lys
   1410 1415 1420
   Glu Gly Thr
   1425
<210> 9
   <211> 45
   <212> DNA
   <213> Artificial Sequence
<220>
   <221> primer_bind
   <222> (1)..(45)
<400> 9
   gctgcaggcg gcatcctaca cctggagctg ctggtggccg tgggc 45
<210> 10
   <211> 20
   <212> DNA
   <213> Artificial Sequence
<220>
   <221> primer_bind
   <222> (1)..(20)
<400> 10
   cggcgggatc ctacacctgg 20
<210> 11
   <211> 20
   <212> DNA
   <213> Artificial Sequence
<220>
   <221> primer_bind
   <222> (1)..(20)
<400> 11
   aatggtgact cccaggtcga 20
<210> 12
   <211> 20
   <212> DNA
   <213> Artificial Sequence
<220>
   <221> primer_bind
   <222> (1)..(20)
<400> 12
   gagcaaattc ctgtactgac 20
<210> 13
   <211> 20
   <212> DNA
   <213> Artificial Sequence
<220>
   <221> primer_bind
   <222> (1)..(20)
<400> 13
   gagcaaattc ctgtactgac 20
<210> 14
   <211> 22
   <212> DNA
   <213> Artificial Sequence
<220>
   <221> primer_bind
   <222> (1)..(22)
<400> 14
   gaccacgcgt atcgacgtcg ac 22
<210> 15
   <211> 21
   <212> DNA
   <213> Artificial Sequence
<220>
   <221> primer_bind
   <222> (1)..(21)
<400> 15
   ctcagggttg atggtctggc t 21
<210> 16
   <211> 30
   <212> DNA
   <213> Artificial Sequence
<220>
   <221> primer_bind
   <222> (1)..(30)
<400> 16
   cgggctgcag gcgggatcct acacctggag 30
<210> 17
   <211> 21
   <212> DNA
   <213> Artificial Sequence
<220>
   <221> primer_bind
   <222> (1) .. (21)
<400> 17
   aatggtgact cccaggtcga g 21
<210> 18
   <211> 20
   <212> DNA
   <213> Artificial Sequence
<220>
   <221> primer_bind
   <222> (1)..(20)
<400> 18
   tggaggtcag caccaacaca 20
<210> 19
   <211> 20
   <212> DNA
   <213> Artificial Sequence
<220>
   <221> primer_bind
   <222> (1)..(20)
<400> 19
   gagttgcctg atggtaaccg 20
<210> 20
   <211> 20
   <212> DNA
   <213> Artificial Sequence
<220>
   <221> primer_bind
   <222> (1) .. (20)
<400> 20
   gagcccttcc gtgggctgca 20
<210> 21
   <211> 21
   <212> DNA
   <213> Artificial Sequence
<220>
   <221> primer_bind
   <222> (1)..(21)
<400> 21
   cgctccctgg tggagctgac c 21
<210> 22
   <211> 20
   <212> DNA
   <213> Artificial Sequence
<220>
   <221> primer_bind
   <222> (1)..(20)
<400> 22
   ctcaccatcg tcctccccat 20
<210> 23
   <211> 21
   <212> DNA
   <213> Artificial Sequence
<220>
   <221> primer_bind
   <222> (1)..(21)
<400> 23
   atcatgaagc gtggagacag c 21
<210> 24
   <211> 20
   <212> DNA
   <213> Artificial Sequence
<220>
   <221> primer_bind
   <222> (1)..(20)
<400> 24
   cctggagggg tccccagatg 20
<210> 25
   <211> 20
   <212> DNA
   <213> Artificial Sequence
<220>
   <221> primer_bind
   <222> (1)..(20)
<400> 25
   tgcagcccac ggaagggctc 20
<210> 26
   <211> 20
   <212> DNA
   <213> Artificial Sequence
<220>
   <221> primer_bind
   <222> (1)..(20)
<400> 26
   cagggctcca ggctgcaggc 20
<210> 27
   <211> 20
   <212> DNA
   <213> Artificial Sequence
<220>
   <221> primer_bind
   <222> (1)..(20)
<400> 27
   aggaagagct gtgtggcctg 20
<210> 28
   <211> 39
   <212> DNA
   <213> Artificial Sequence
<220>
   <221> primer_bind
   <222> (1)..(39)
<400> 28
   gacgcggccc agccggccgc tgcaggcggc atcctacac 39
<210> 29
   <211> 34
   <212> DNA
   <213> Artificial Sequence
<220>
   <221> primer_bind
   <222> (1)..(34)
<400> 29
   ggccctcgag cggttccttc ctttcccttc cagg 34
<210> 30
   <211> 35
   <212> DNA
   <213> Artificial Sequence
<220>
   <221> primer_bind
   <222> (1)..(35)
<400> 30
   agcggtctct atggctgcag gcggcatcct acacc 35
<210> 31
   <211> 30
   <212> DNA
   <213> Artificial Sequence
<220>
   <221> primer_bind
   <222> (1) .. (30)
<400> 31
   agcctcgagc tggccagaca cggaacaaat 30
<210> 32
   <211> 29
   <212> DNA
   <213> Artificial Sequence
<220>
   <221> primer_bind
   <222> (1)..(29)
<400> 32
   ggcgaattca tgcaccagcg tcacccccg 29
<210> 33
   <211> 21
   <212> DNA
   <213> Artificial Sequence
<220>
   <221> primer_bind
   <222> (1)..(21)
<400> 33
   acagcattaa actaagccgc c 21
<210> 34
   <211> 40
   <212> DNA
   <213> Artificial Sequence
<220>
   <221> primer_bind
   <222> (1)..(40)
<400> 34
   gatcgaattc gccggccacc atgcaccagc gtcacccccg 40
<210> 35
   <211> 21
   <212> DNA
   <213> Artificial Sequence
<220>
   <221> primer_bind
   <222> (1)..(21)
<400> 35
   cggataacaa tttcacacag g 21